# EUROPÄISCHE PATENTSCHRIFT

(11) **EP 1 423 398 B1**
(45) Veröffentlichungstag und Bekanntmachung des Hinweises auf die Patenterteilung: **17.05.2006**
(21) Anmeldenummer: 02758461.4
(22) Anmeldetag: 13.08.2002
(51) Int. Cl.: C07F 9/6574

(54) **NEUE PHOSPHITVERBINDUNGEN UND NEUE PHOSPHITMETALLKOMPLEXE**
NOVEL PHOSPHITE COMPOUNDS AND NOVEL PHOSPHITE METAL COMPLEXES
NOUVEAUX COMPOSES DE PHOSPHITE ET NOUVEAUX COMPLEXES METALLIQUES DE PHOSPHITE

(30) Priorität: 16.08.2001 DE 10140086
(43) Veröffentlichungstag der Anmeldung: 02.06.2004
(60) Teilanmeldung: 05105175.3
(73) Patentinhaber: Oxeno Olefinchemie GmbH, 45772 Marl (DE)
(72) Erfinder: SELENT, Detlef,Dr., 10318 Berlin (DE); BÖRNER, Armin, 18059 Rostock (DE); BORGMANN, Cornelia, 45657 Recklinghausen (DE); HESS, Dieter, 45770 Marl (DE); WIESE, Klaus-Diether, 45721 Haltern (DE)
(74) Vertreter: Lang, Arne
(86) Internationale Anmeldenummer: PCT/EP2002/009050
(87) Internationale Veröffentlichungsnummer: WO 2003/016321

(56) Entgegenhaltungen:
- EP-A- 1 201 675
- NL-A- 6 514 392
- SELENT D ET AL: "NEW PHOSPHORUS LIGANDS FOR THE RHODIUM-CATALYZED ISOMERIZATION/HYDROFORMYLATION OF INTERNAL OCTENES" ANGEWANDTE CHEMIE. INTERNATIONAL EDITION, VERLAG CHEMIE. WEINHEIM, DE, Bd. 40, Nr. 9, 4. Mai 2001 (2001-05-04), Seiten 1696-1698, XP001009251 ISSN: 0570-0833
- SIEDENTOP T ET AL: "SYNTHESE UND KOMPLEXIERUNG PHOSPHORYLIERTER SPACER-MODIFIZIERTER GLUCOSEDERIVATE SYNTHESIS AND COMPLEXATION OF SPACER MODIFIED PHOSPHORYLATED GLUCOSE DERIVATIVES" ZEITSCHRIFT FUR NATURFORSCHUNG, TEIL B: ANORGANISCHE CHEMIE, ORGANISCHE CHEMIE, VERLAG DER ZEITSCHRIFT FUR NATURFORSCHUNG. TUBINGEN, DE, Bd. 55, Nr. 10, 2000, Seiten 956-960, XP001027205 ISSN: 0932-0776
- SHADID B ET AL: "The Synthesis of Cytokinin Phophatases" TETRAHEDRON, ELSEVIER SCIENCE PUBLISHERS, AMSTERDAM, NL, Bd. 46, Nr. 3, 1990, Seiten 901-912, XP002186808 ISSN: 0040-4020
- DATABASE CA [Online] CHEMICAL ABSTRACTS SERVICE, COLUMBUS, OHIO, US; MIRONOV, V. F. ET AL: "Behavior of fluoroalkyl phosphites in reactions with sulfur chlorides" retrieved from STN Database accession no. 122:187680 XP002216984 & ZHURNAL OBSHCHEI KHIMII (1994), 64(8), 1358-61,
- DATABASE CROSSFIRE BEILSTEIN [Online] Beilstein Institut zur Förderung der Chemischen Wissenschaften, Frankfurt am Main, DE; Database accession no. BRN 4874849; BRN 4886067 XP002216985 & MIRONOV, V.F.: IZV. AKSD. NAUK. SSSR SER KHIM.; RU, Nr. 3, 1993, Seiten 565-567,
- DATABASE CA [Online] CHEMICAL ABSTRACTS SERVICE, COLUMBUS, OHIO, US; LUDWIG, JANOS ET AL: "Stereospecific synthesis of guanosine 5'-O-(1,2-dithiotriphosphates)" retrieved from STN Database accession no. 115:183763 XP002216986 & JOURNAL OF ORGANIC CHEMISTRY (1991), 56(20), 5860-5,
- DATABASE CROSSFIRE BEILSTEIN [Online] Beilstein Institut zur Förderung der Chemischen Wissenschaften, Frankfurt am Main, DE; Database accession no. BRN 8602957 XP002216987 & SCHOETZAU, TH. ET AL.: J. CHEM. SOC. PERKIN TRANS. 1, Nr. 9, 2000, Seiten 1411-1416,
- DATABASE CROSSFIRE BEILSTEIN [Online] Beilstein Institut zur Förderung der Chemischen Wissenschaften, Frankfurt am Main, DE; Database accession no. BRN 8519330 BRN 8532363 BRN 8530950 XP002216988 & SCHOETZAU, TH. ET AL.: SYNTHESIS, Bd. 5, 2000, Seiten 707-713,
- DATABASE CROSSFIRE BEILSTEIN [Online] Beilstein Institut zur Förderung der Chemischen Wissenschaften, Frankfurt am Main, DE; Database accession no. BRN8530185 BRN 8524530 XP002216989 & WOJCZEWSKI, CH. ET AL.: SYNTHESIS, Nr. 1, 2000, Seiten 149-153,
- DATABASE CROSSFIRE BEILSTEIN [Online] Beilstein Institut zur Förderung der Chemischen Wissenschaften, Frankfurt am Main, DE; Database accession no. BRN 8592202 BRN 8605310 XP002216990 & WOJCZEWSKI, CH. ET AL.: HELV. CHIM. ACTA, Bd. 83, Nr. 6, 2000, Seiten 1268-1277,
- DATABASE CROSSFIRE BEILSTEIN [Online] Beilstein Institut zur Förderung der Chemischen Wissenschaften, Frankfurt am Main, DE; Database accession no. BRN 5446652 XP002216991 & MIRONOV, V. F. ET AL.: ZH. OBSHCH. KIM. RU, Bd. 61, Nr. 10, 1991, Seiten 2150-2154,
- DATABASE CA [Online] CHEMICAL ABSTRACTS SERVICE, COLUMBUS, OHIO, US; KIRPICHNIKOV, P. A. ET AL: "Phosphorous acid esters as colorless stabilizers of low-pressure polyethylene" retrieved from STN Database accession no. 73:15657 XP002216992 in der Anmeldung erwähnt & VYSOKOMOL. SOEDIN., SER. B (1970), 12(3), 189-92,
- DATABASE CROSSFIRE BEILSTEIN [Online] Beilstein Institut zur Förderung der Chemischen Wissenschaften, Frankfurt am Main, DE; Database accession no. BRN 2660456; BRN 2662683; BRN 2662684; BRN 2664377 XP002226542 & NESTEROW, SABIROWA: J. GEN. CHEM. USSR (ENGL. TRANSL.), Bd. 35, 1965, Seite 1967
- DATABASE CROSSFIRE BEILSTEIN [Online] Beilstein Institut zur Förderung der Chemischen Wissenschaften, Frankfurt am Main, DE; Database accession no. BRN 2657140; Reaction ID 5068507 XP002226543 & MUNOZ, A. ET AL.: J. ORG. CHEM., Bd. 61, Nr. 17, 1996, Seiten 6015-6017,
- DATABASE CROSSFIRE BEILSTEIN [Online] Beilstein Institut zur Förderung der Chemischen Wissenschaften, Frankfurt am Main, DE; Database accession no. BRN 2665957 XP002226544 & CADE, J.A. ET AL.: J. CHEM. SOC., 1960, Seiten 1249-1253,

## Beschreibung

Die vorliegende Erfindung betrifft neue Phosphitverbindungen und neue Phosphitmetallkomplexe, sowie deren Verwendung in katalytischen Reaktionen.

Die Reaktionen zwischen Olefinverbindungen. Kohlenmonoxid und Wasserstoff in Gegenwart eines Katalysators zu den um ein C-Atom reicheren Aldehyden ist als Hydroformylierung (0xierung) bekannt. Als Katalysatoren in diesen Reaktionen werden häufig Verbindungen der Übergangsmetalle der 8. bis 10. Gruppe des Periodensystems der Elemente verwendet, insbesondere Verbindungen des Rhodiums und des Kobalts. Die Hydroformylierung mit Rhodiumverbindungen bietet im Vergleich zur Katalyse mit Kobaltverbindungen in der Regel den Vorteil höherer Selektivität und ist damit meistens wirtschaftlicher. Bei der durch Rhodium katalysierten Hydroformylierung werden zumeist Komplexe eingesetzt, die aus Rhodium und bevorzugt aus trivalenten Phosphorverbindungen als Liganden bestehen. Bekannte Liganden sind beispielsweise Verbindungen aus den Klassen der Phosphine, Phosphite und Phosphonite. Eine Übersicht über Hydroformylierung von Olefinen findet sich in B. CORNILS, W. A. HERRMANN. "Applied Homogeneous Catalysis with Organometallic Compounds", Vol. 1&2. VCH. Weinheim, New York, 1996.

Jedes Katalysatorsystem (Kobalt oder Rhodium) hat seine spezifischen Vorzüge. Je nach Einsatzstoff und Zielprodukt werden unterschiedliche Katalysatorsysteme verwendet. Arbeitet man mit Rhodium und Triphenylphosphin, lassen sich α-Olefine bei niedrigeren Drücken hydroformylieren. Als phosphorhaltiger Ligand wird in der Regel Triphenylphosphin im Überschuss verwendet, wobei ein hohes Ligand/Rhodium-Verhältnis erforderlich ist, um die Selektivität der Reaktion zum kommerziell erwünschten n-Aldehydprodukt zu erhöhen.

US-A-4,694,109 und US-A-4,879,416 betreffen Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten erreicht.

In WO-A-95/30680 werden zweizähnige Phosphinliganden und ihr Einsatz in der Katalyse, unter anderem auch in Hydroformylierungsreaktionen, beschrieben.

Ferrocenverbrückte Bisphosphine werden beispielsweise in US-A-4,169,861, US-A-4,201,714 und US-A-4,193,943 als Liganden für Hydroformylierungen offenbart.

Der Nachteil von zweizähnigen Phosphinliganden ist die relativ aufwendige Herstellung. Daher ist es oftmals nicht rentabel, solche Systeme in technischen Prozessen einzusetzen.

Rhodium-Monophosphit-Komplexe sind geeignete Katalysatoren für die Hydroformylierung von verzweigten Olefinen mit innenständigen Doppelbindungen, jedoch ist die Selektivität für endständig hydroformylierte Verbindungen gering. Aus EP-A-0 155 508 ist die Verwendung von bisarylensubstituierten Monophosphiten bei der rhodiumkatalysierten Hydroformylierung von sterisch gehinderten Olefinen, z. B. Isobuten bekannt.

Rhodium-Phosphit-Komplexe katalysieren die Hydroformylierung von linearen Olefinen mit end- und innenständigen Doppelbindungen, wobei überwiegend endständig hydroformylierte Produkte entstehen, dagegen werden verzweigte Olefine mit innenständigen Doppelbindungen nur in geringem Maße umgesetzt. Diese Phosphite ergeben bei ihrer Koordination an ein Übergangsmetallzentrum Katalysatoren von gesteigerter Aktivität, doch ist das Standzeitverhalten dieser Katalysatorsysteme, unter anderem wegen der Hydrolyseempfindlichkeit der Phosphitliganden, unbefriedigend. Durch den Einsatz von substituierten Bisaryldiolen als Edukte für die Phosphitliganden, wie in EP-A-0 214 622 oder EP-A-0 472 071 beschrieben, konnten erhebliche Verbesserungen erreicht werden.

Der Literatur zufolge sind die Rhodiumkomplexe dieser Liganden äußerst aktive Hydroformylierungskatalysatoren für α-Olefine. In US-A-4,668,651, US-A-4,748,261 und US-A-4,885,401 werden Polyphosphitliganden beschrieben, mit denen α-Olefine, aber auch 2-Buten mit hoher Selektivität zu den terminal hydroformylierten Produkten umgesetzt werden können. In US-A-5,312,996 werden zweizähnige Liganden dieses Typs auch zur Hydroformylierung von Butadien eingesetzt.

Obgleich die genannten Phosphite gute Komplexliganden für Rhodium-Hydroformylierungskatalysatoren sind, ist es wünschenswert, weitere leicht herstellbare Phosphite zur weiteren Verbesserung ihrer Wirksamkeit beispielsweise in der Hydroformylierung aufzufinden.

Phosphite mit Salicylsäure-Bausteinen gemäß Formel Δ 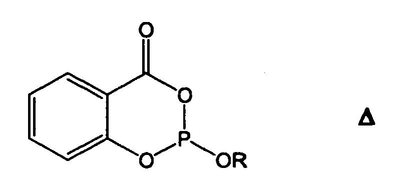 mit R = Alkyl, Aryl, Aralkyl, Alkenyl. Cycloalkyl, Acyl, COPh wurden in den Patenten JP 06025493, JP 2000038487 und JP 10081801 als Stabilisatoren für Kunststoffe beschrieben.
P. A. Kirpichnikov et al. zeigen die stabilisierenden Eigenschaften von Phosphiten mit Salicylsäure-Bausteinen im russischen Journal Vysokomol. Soedin., Ser. B (1970). 12 (3). 189-192.

Es wurde überraschend gefunden, dass Phosphite gemäß Formel I 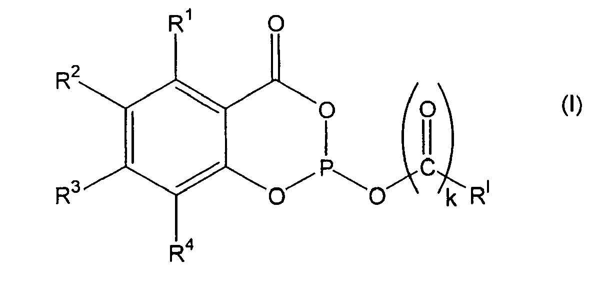 wobei R¹ ausgewählt ist aus einwertigen unsubstituierten aliphatischen, alicyclischen, gemischt aliphatisch-alicyclischen, gemischt aliphatisch-aromatischen, heterocyclischen, und gemischt aliphatisch-heterocyclischen Kohlenwasserstoffresten mit 1 bis 50 Kohlenstoffatomen, oder aus einwertigen aromatischen Kohlenwasserstoffresten mit 1 bis 50 Kohlenstoffatomen, die unsubstituiert oder mit mindestens einem Rest, ausgewählt aus alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 25 Kohlenstoffatomen, F, Cl, Br, I, -CF₃, -CH₂(CF₂)ⱼCF₃ mit j = 0 - 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰, oder -N=CR⁹R¹⁰, substituiert sind, oder aus einwertigen heteroaromatischen Kohlenwasserstoffresten mit 1 bis 50 Kohlenstoffatomen, die unsubstituiert oder mit mindestens einem Rest, ausgewählt aus aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 25 Kohlenstoffatomen, F, Cl, Br, I, -CF₃, -CH₂(CF₂)ⱼCF₃ mit j = 0 - 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰, oder -N=CR⁹R¹⁰, substituiert sind, R¹, R², R³, und R⁴ jeweils unabhängig voneinander ausgewählt sind aus einwertigen substituierten oder unsubstituierten aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 50 Kohlenstoffatomen. H. F, Cl, Br, I, -CF₃, -CH₂(CF₂)ⱼCF₃ mit j = 0 - 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰, -N=CR⁹R¹⁰, wobei R⁹ und R¹⁰ unabhängig voneinander aus H, einwertigen substituierten oder unsubstituierten aliphatischen und aromatischen Kohlenwasserstoffresten mit 1 bis 25 Kohlenstoffatomen ausgewählt sind und M ein Alkalimetall-, formal ein halbes Erdalkalimetall-, Ammonium- oder Phosphoniumion ist,
oder benachbarte Reste R¹ bis R⁴ zusammen ein kondensiertes substituiertes oder unsubstituiertes aromatisches, heteroaromatisches, aliphatisches, gemischt aromatisch-aliphatisches oder gemischt heteroaromatisch-aliphatisches Ringsystem ausbilden;
und k = 0 ist,
wobei wenn R¹, R³, R³ und R⁴ jeweils ein H-Atom sind und k = 0 ist, R¹ keine Alkyl-, Aryl-, Aralkyl-, Alkenyl-, oder Cycloalkylgruppe ist, oder gemäß der Formeln 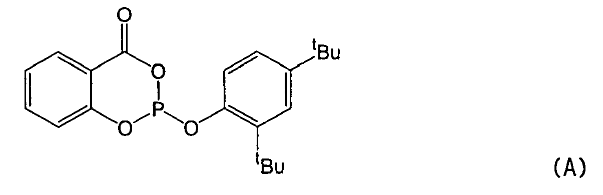 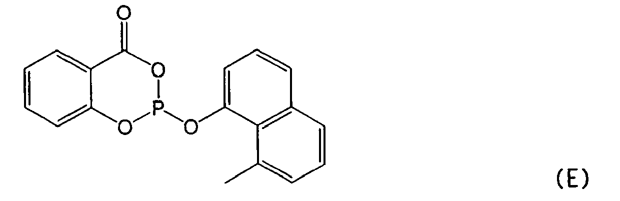 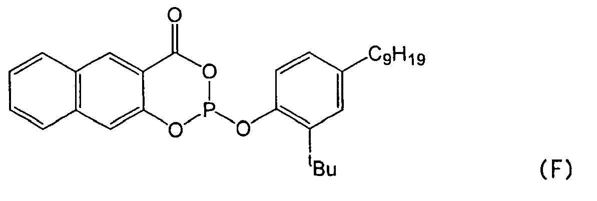 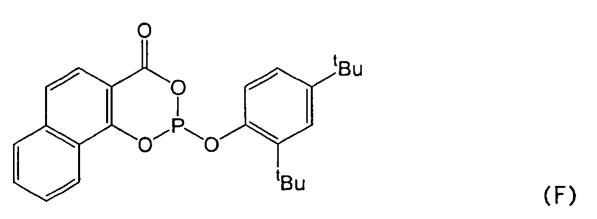 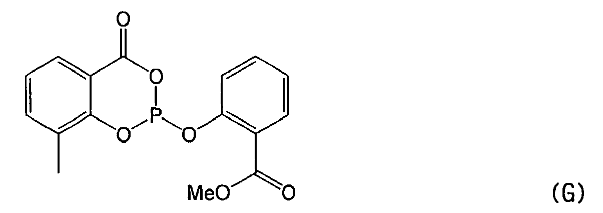 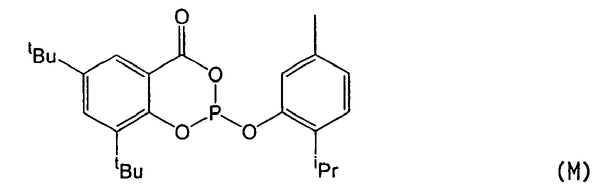 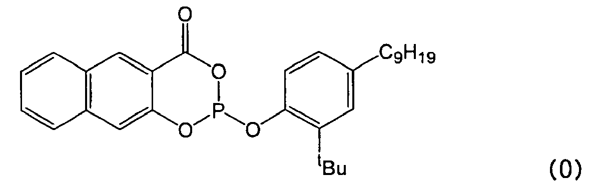 in der Katalyse verwendet werden können.

Ebenfalls wurde gefunden, dass Phosphitmetallkomplexe, enthaltend ein Metall der 4., 5., 6., 7., 8., 9. oder 10. Gruppe des Periodensystems der Elemente und ein oder mehrere der Phosphite gemäß Formel I, 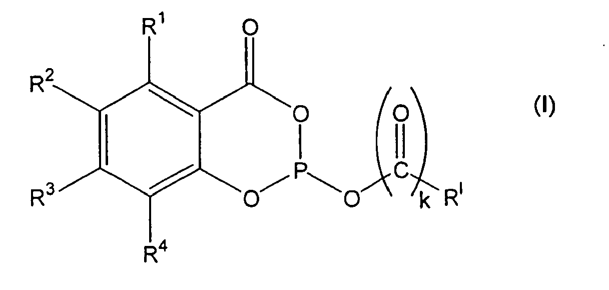 wobei R^{l} ausgewählt ist aus einwertigen unsubstituierten aliphatischen, alicyclischen, gemischt aliphatisch-alicyclischen, gemischt aliphatisch-aromatischen, heterocyclischen, und gemischt aliphatisch-heterocyclischen Kohlenwasserstoffresten mit 1 bis 50 Kohlenstoffatomen oder aus einwertigen aromatischen und heteroaromatischen Kohlenwasserstoffresten mit 1 bis 50 Kohlenstoffatomen, die unsubstituiert oder mit mindestens einem Rest, ausgewählt aus aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 25 Kohlenstoffatomen. F, Cl, Br, I. -CF₃, -CH₂(CF₂)ⱼCF₃ mit j = 0 - 9, -OR⁹, -COR⁹, -CO₂R⁹, -CO₂M, -SR⁹, -SO₂R⁹, -SOR⁹. -SO₃R⁹, -SO₃M, -SO₂NR⁹R¹⁰, -NR⁹R¹⁰, oder -N=CR⁹R¹⁰, substituiert sind,

R¹, R², R³, und R⁴ jeweils unabhängig voneinander ausgewählt sind aus einwertigen substituierten oder unsubstituierten aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 50 Kohlenstoffatomen.H, F, Cl , Br, I, -CF₃, -CH₂(CF₂)ⱼCF₃ mit j = 0 - 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰, -N=CR⁹R¹⁰, wobei R⁹ und R¹⁰ unabhängig voneinander aus H, einwertigen substituierten oder unsubstituierten aliphatischen und aromatischen Kohlenwasserstoffresten mit 1 bis 25 Kohlenstoffatomen ausgewählt sind und M ein Alkalimetall-, formal ein halbes Erdalkalimetall-, Ammonium- oder Phosphoniumion ist,
oder benachbarte Reste R¹ bis R⁴ zusammen ein kondensiertes substituiertes oder unsubstituiertes aromatisches, heteroaromatisches, aliphatisches, gemischt aromatisch-aliphatisches oder gemischt heteroaromatisch-aliphatisches Ringsystem ausbilden; und k = 0 ist, in der Katalyse verwendet werden können.

Die vorliegende Erfindung ist vorzugsweise auf die Verwendung der Phosphite bzw. der Phosphitmetallkomplexe in der homogenen Katalyse, insbesondere bei der Hydroformylierung von Olefinen, gerichtet. Die Erfindung betrifft auch ein Verfahren zur Hydroformylierung von Olefinen.

Ein weiterer Aspekt der vorliegenden Erfindung sind die oben genannten Phosphitmetallkomplexe.

In einem bevorzugten Phosphit ist der Rest R^{l} ausgewählt aus Aromaten oder Heteroaromaten, die ankondensierte aromatische, heteroaromatische und/oder aliphatische Ringe aufweisen, die unsubstituiert oder mit mindestens einem Rest, ausgewählt aus aliphatischen, alicyclischen, aromatischen, heteroaromatischen, gemischt aliphatisch-alicyclischen, gemischt aliphatisch-aromatischen, heterocyclischen, gemischt aliphatisch heterocyclischen Kohlenwasserstoffresten mit 1 bis 25 Kohlenstoffatomen, F, Cl, Br, I, -CF₃, -CH₂(CF₂)ⱼCF₃ mit j = 0 - 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰, oder -N=CR⁹R¹⁰, substituiert sind, wobei R⁹, R¹⁰ und M wie zuvor definiert sind. Weiterhin ist es bevorzugt, ein Phosphit zu verwenden, dessen Reste R¹ bis R⁴ zusammen ein kondensiertes aromatisches, heteroaromatisches, aliphatisches, gemischt aromatisch-aliphatisches oder gemischt heteroaromatisch-aliphatisches Ringsystem ausbilden, das unsubstituiert ist oder mit mindestens einem Rest, ausgewählt aus aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 50 Kohlenstoffatomen, F, C1. Br, I, -CF₃, -CH₂(CF₂)ⱼCF₃ mit j = 0 - 9. -OR⁹. -COR⁹. -CO₂R⁹, -CO₂M, -SR⁹, -SO₂R⁹, -SOR⁹, -SO₃R⁹, -SO₃M. -SO₂NR⁹R¹⁰. -NR⁹R¹⁰, oder -N=CR⁹R¹⁰. substituiert ist, wobei R⁹, R¹⁰ und M wie zuvor definiert sind.

Repräsentative Phosphitliganden gemäß der Formel I zur erfindungsgemäßen Verwendung sind:

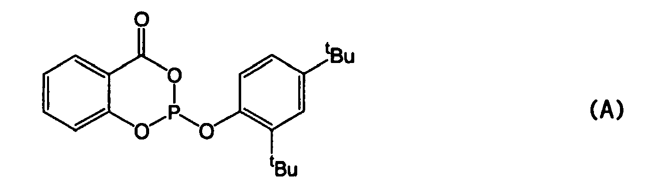

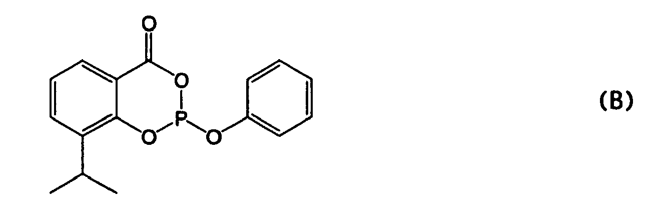

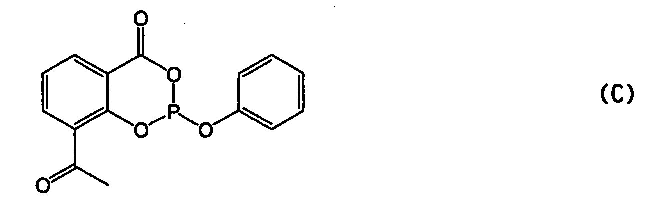

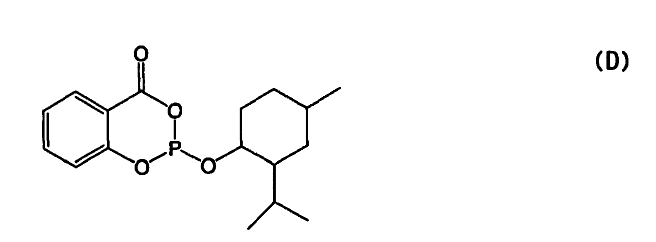

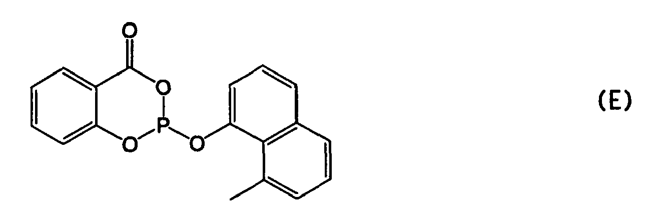

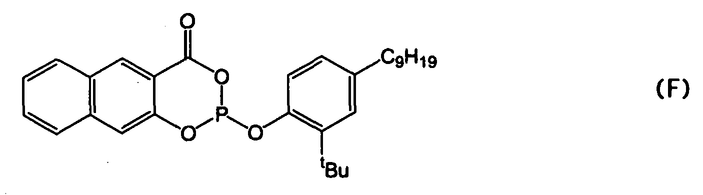

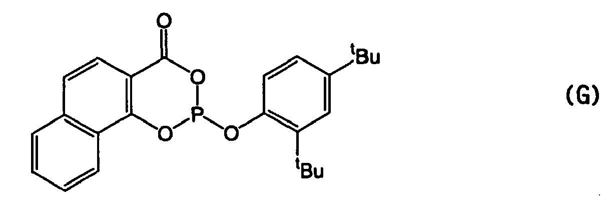

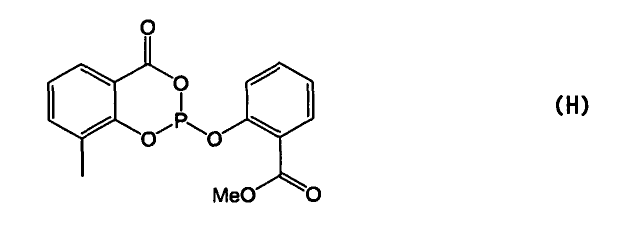

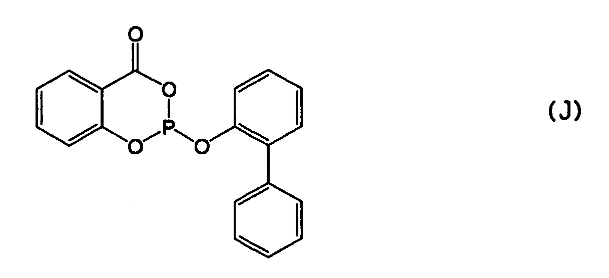

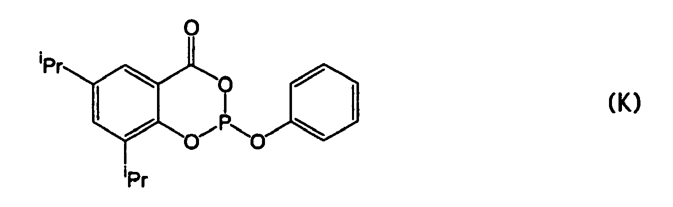

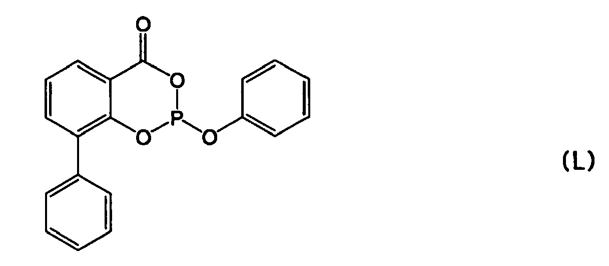

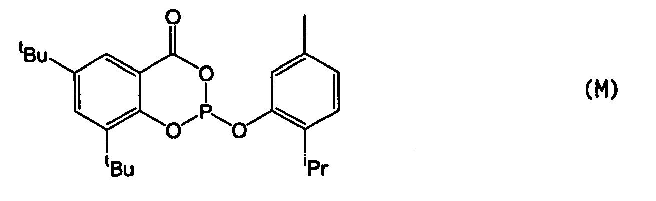

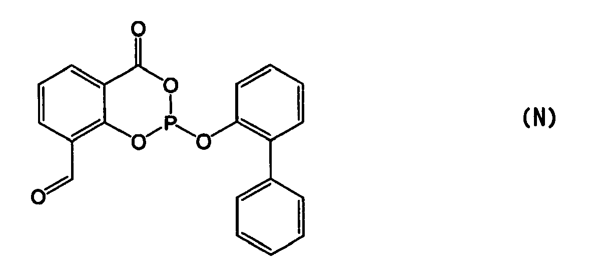

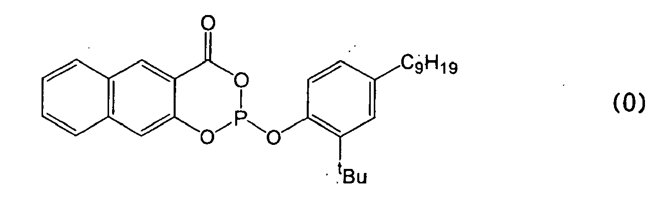

Die erfindungsgemäßen Phosphite zur Verwendung können durch eine Folge von Reaktionen von Phosphorhalogeniden mit Alkoholen und/oder α-Hydroxyarylcarbonsäuren, bei denen Halogenatome am Phosphor gegen Sauerstoffgruppen ausgetauscht werden, hergestellt werden. Das grundsätzliche Vorgehen wird beispielhaft an einem Weg zu Verbindungen gemäß der allgemeinen Formel I illustriert:

In einem ersten Schritt wird eine α-Hydroxyarylcarbonsäure mit einem Phosphortrihalogenid PX₃, wie etwa PCl₃, PBr₃ und PJ₃, vorzugsweise Phosphortrichlorid PCl₃, in Gegenwart einer Base, die vorzugsweise in äquivalenten oder katalytischen Mengen eingesetzt wird, zu einem Halogendioxaphosphorinon (1) umgesetzt.

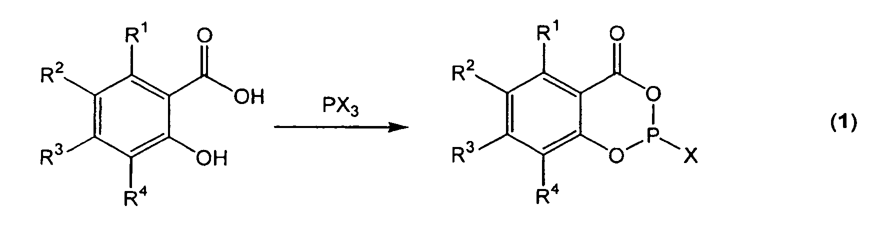

In einem zweiten Reaktionsschritt wird aus dem Halogendioxaphosphorinon (1) durch Reaktion mit einem Alkohol HO-R^{l} in Gegenwart einer Base, die vorzugsweise in äquivalenten oder katalytischen Mengen eingesetzt wird, das gewünschte Phosphit gemäß Formel (I) erhalten, in dem R^{l} = R" ist. Im Falle der Umsetzung mit einem Alkohol ist k in dem Phosphit gemäß Formel (I) gleich 0.

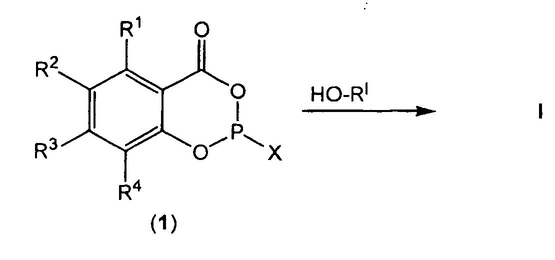

Die Reste R¹ bis R⁴ und R^{l} haben die zuvor angegebenen Bedeutungen. Da die eingesetzten Alkohole und ihre Folgeprodukte häufig fest sind, werden die Umsetzungen im Allgemeinen in Lösungsmitteln durchgeführt. Als Solventien werden nichtprotische Lösungsmittel, die weder mit den Alkoholen noch mit den Phosphorverbindungen reagieren, verwendet. Geeignete Lösungsmittel sind beispielsweise Tetrahydrofuran, Ether wie Diethylether oder MTBE (Methyl-tertiärbutylether) oder aromatische Kohlenwasserstoffe wie Toluol.

Bei der Umsetzung von Phosphorhalogeniden mit Alkoholen entsteht Halogenwasserstoff, der durch zugegebene Basen in äquivalenten oder in katalytischen Mengen gebunden wird. Beispielsweise werden dafür tertiäre Amine, wie Triethylamin, Pyridin oder N-Methylpyrrolidinon, eingesetzt. Teilweise ist es auch sinnvoll, die Alkohole vor der Reaktion in Metallalkoholate zu überführen, zum Beispiel durch Reaktion mit Natriumhydrid oder Butyllithium.

Die Phosphite sind geeignete Liganden zur Komplexierung von Metallen der 4., 5., 6., 7., 8., 9. oder 10. Gruppe des Periodensystems der Elemente. Die Komplexe können ein oder mehrere Phosphitliganden und gegebenenfalls weitere Liganden enthalten und sind als Katalysatoren geeignet, vorzugsweise bei der homogenen Katalyse. Beispiele für geeignete Metalle sind Rhodium, Kobalt. Iridium, Nickel, Palladium, Platin. Eisen. Ruthenium, Osmium, Chrom, Molybdän und Wolfram. Insbesondere mit Metallen der 8., 9. oder 10. Gruppe können die resultierenden Komplexe als Katalysatoren für Hydroformylierungs-Carbonylierungs-, Hydrierungs- und Hydrocyanierungsreaktionen verwendet werden, besonders bevorzugt sind Rhodium, Kobalt, Nickel, Platin und Ruthenium. Beispielsweise ergeben sich in Hydroformylierungsreaktionen besonders bei Einsatz von Rhodium als Katalysatormetall hohe katalytische Aktivitäten. Die Katalysatormetalle kommen in Form von Salzen oder Komplexen zum Einsatz, im Falle von Rhodium z.B. Rhodiumcarbonyle, Rhodiumnitrat, Rhodiumchlorid. Rh(CO)₂(acac) (acac = Acetylacetonat), Rhodiumacetat, Rhodiumoctanoat oder Rhodiumnonanoat.

Aus den Phosphitliganden und dem Katalysatormetall bildet sich unter Reaktionsbedingungen die aktive Katalysatorspezies für die homogene Katalyse, etwa bei der Hydroformylierung bei Kontakt mit Synthesegas ein Carbonylhydridphosphit-Komplex. Die Phosphite und gegebenenfalls weitere Liganden können in freier Form zusammen mit dem Katalysatormetall (als Salz oder Komplex) in die Reaktionsmischung gegeben werden, um die aktive Katalysatorspezies in situ zu erzeugen. Es ist weiterhin auch möglich, einen Phosphitmetallkomplex, der die o. g. Phosphitliganden und das Katalysatormetall enthält, als Precursor für den eigentlichen katalytisch aktiven Komplex einzusetzen. Diese Phosphitmetallkomplexe werden hergestellt, indem man man das entsprechende Katalysatormetall der 4. bis 10. Gruppe in elementarer Form oder in Form einer chemischen Verbindung mit dem Phosphitliganden umsetzt.

Als zusätzliche, im Reaktionsgemisch vorhandene Liganden können phosphorhaltige Liganden, vorzugsweise Phosphine. Bisphosphite, Phosphonite oder Phosphinite eingesetzt werden.

Beispiele für solche Liganden sind:
Phosphine: Triphenylphosphin, Tris(p-tolyl)phosphin, Tris(m-tolyl)phosphin, Tris(o-tolyl)phosphin, Tris(p-methoxyphenyl)phosphin. Tris(p-dimethylaminophenyl)phosphin, Tricyclohexylphosphin, Tricyclopentylphosphin, Triethylphosphin, Tri-(1-naphthyl)phosphin, Tribenzylphosphin, Tri-n-butylphosphin, Tri-t-butylphosphin.
Phosphite: Trimethylphosphit, Triethylphosphit, Tri-n-propylphosphit, Tri-i-propylphosphit, Tri-n-butylphosphit, Tri-i-butylphosphit, Tri-t-butylphosphit, Tris(2-ethylhexyl)phosphit, Triphenylphosphit, Tris(2.4-di-t-butylphenyl)phosphit, Tris(2-t-butyl-4-methoxyphenyl)phosphit, Tris(2-t-butyl-4-methylphenyl)phosphit, Tris(p-kresyl)phosphit. Außerdem sind sterisch gehinderte Phosphitliganden, wie sie unter anderem in EP-A-155 508, US-A-4, 668,651, US-A-4,748,261, US-A-4,769,498, US-A-4,774,361, US-A-4,835,299, US-A-4,885,401, US-A-5,059,710, US-A-5,113,022, US-A-5,179,055, US-A-5,260,491, US-A-5,264,616, US-A-5,288,918, US-A-5,360,938, EP-A-472 071, EP-A-518 241 und WO-A-97/20795 beschrieben werden, geeignete Liganden.
Phosphonite: Methyldiethoxyphosphin. Phenyldimethoxyphosphin, Phenyldiphenoxyphosphin, 2-Phenoxy-2H-di benz[c,e][1,2]oxaphosphori n und dessen Derivate, in denen die Wasserstoffatome ganz oder teilweise durch Alkyl- und/oder Arylreste oder Halogenatome ersetzt sind und Liganden, die in W0-A-98/43935. JP-A-09-268152 und DE-A-198 10 794 und in den deutschen Patentanmeldungen DE-A-199 54 721 und DE-A-199 54 510 beschrieben sind.

Gängige Phosphinitliganden sind unter anderem in US-A-5.710.344. WO-A-95/06627. US-A-5,360,938 oder JP-A-07-082281 beschrieben. Beispiele hierfür sind Diphenyl(phenoxy)phosphin und dessen Derivate, in denen die Wasserstoffatome ganz oder teilweise durch Alkyl- und/oder Arylreste oder Halogenatome ersetzt sind, Diphenyl(methoxy)phosphin und Diphenyl(ethoxy)phosphin.

Die Phosphite bzw. Phosphitmetallkomplexe können in Verfahren zur Hydroformylierung von Olefinen, bevorzugt mit 2 bis 25 Kohlenstoffatomen, zu den entsprechenden Aldehyden eingesetzt werden. Hierbei werden bevorzugt Phosphitkomplexe mit Metallen der 8. Nebengruppe als Katalysator-Vorläufer verwendet.

Im Allgemeinen werden 1 bis 500 mol, vorzugsweise 1 bis 200 mol, bevorzugter 2 bis 50 mol des erfindungsgemäßen Phosphits pro mol Metall der 8. Nebengruppe eingesetzt.

Frischer Phosphitligand kann zu jedem Zeitpunkt der Reaktion zugesetzt werden, um die Konzentration an freiem Liganden konstant zu halten.

Die Konzentration des Metalls im Reaktionsgemisch liegt im Bereich von 1 ppm bis 1000 ppm, vorzugsweise im Bereich von 5 ppm bis 300 ppm, bezogen auf das Gesamtgewicht der Reaktionsmischung.

Die mit den erfindungsgemäßen Phosphiten bzw. den entsprechenden Metallkomplexen durchgeführten Hydroformylierungsreaktionen erfolgten nach bekannten Vorschriften, wie z. B. in J. FALBE, "New Syntheses with Carbon Monoxide". Springer Verlag, Berlin, Heidelberg, New York, Seite 95 ff., (1980) beschrieben. Die Olefinverbindung(en) wird (werden) in Gegenwart des Katalysators mit einem Gemisch aus CO und H₂ (Synthesegas) zu den um ein C-Atom reicheren Aldehyden umgesetzt.

Die Reaktionstemperaturen für ein Hydroformylierungsverfahren mit den erfindungsgemäßen Phosphiten bzw. Phosphitmetallkomplexen als Katalysator liegen vorzugsweise zwischen 40 °C und 180 °C und bevorzugter zwischen 75 °C und 140 °C. Die Drücke, unter denen die Hydroformylierung abläuft, betragen vorzugsweise 1 - 300 bar Synthesegas und bevorzugter 10 - 64 bar. Das Molverhältnis zwischen Wasserstoff und Kohlenmonoxid (H₂/CO) im Synthesegas beträgt vorzugsweise 10/1 bis 1/10 und bevorzugter 1/1 bis 2/1.

Der Katalysator bzw. der Ligand ist homogen im Hydroformylierungsgemisch, bestehend aus Edukten (Olefinen und Synthesegas) und Produkten (Aldehyden, Alkoholen, im Prozess gebildete Hochsieder), gelöst. Optional kann zusätzlich ein Lösungsmittel verwendet werden.

Aufgrund ihres relativ hohen Molekulargewichtes besitzen die erfindungsgemäßen Phosphite eine geringe Flüchtigkeit. Sie können daher einfach von den leichter flüchtigen Reaktionsprodukten abgetrennt werden. Sie sind in den gängigen organischen Solventien ausreichend gut löslich.

Die Edukte für die Hydroformylierung sind Olefine oder Gemische von Olefinen mit 2 bis 25 Kohlenstoffatomen mit end- oder innenständiger C=C-Doppelbindung. Sie können geradkettig, verzweigt oder von cyclischer Struktur sein und auch mehrere olefinisch ungesättigte Gruppen aufweisen. Beispiele sind Propen: 1-Buten, cis-2-Buten, trans-2-Buten. Isobuten, Butadien. Mischungen der C₄-Olefine: C₅-Olefine wie 1-Penten, 2-Penten. 2-Methylbuten-1, 2-Methylbuten-2, 3-Methylbuten-1: C₆-Olefine wie 1-Hexen, 2-Hexen. 3-Hexen, das bei der Dimerisierung von Propen anfallende C₆-Olefingemisch (Dipropen): C₇-Olefine wie 1-Hepten, weitere n-Heptene. 2- Methyl-1-hexen. 3-Methyl-1-hexen: C₈-Olefine wie 1-Octen, weitere n-Octene, 2-Methylheptene, 3-Methylheptene, 5-Methylhepten-2, 6-Methylhepten-2, 2-Ethylhexen-1, das bei der Dimerisierung von Butenen anfallende isomere C₈-Olefingemisch (Dibuten): C₉-Olefine wie 1-Nonen, weitere n-Nonene, 2-Methyloctene. 3-Methyloctene, das bei der Trimerisierung von Propen anfallende C₉-Olefingemisch (Tripropen): C₁₀-Olefine wie n-Decene. 2-Ethyl-1-octen: C₁₂-Olefine wie n-Dodecene, das bei der Tetramerisierung von Propen oder der Trimerisierung von Butenen anfallende C₁₂-Olefingemisch (Tetrapropen oder Tributen). C₁₄-Olefine wie n-Tetradecene, C₁₆-Olefine wie n-Hexadecene, das bei der Tetramerisierung von Butenen anfallende C₁₆-Olefingemisch (Tetrabuten) sowie durch Cooligomerisierung von Olefinen mit unterschiedlicher C-Zahl (bevorzugt 2 bis 4) hergestellte Olefingemische, gegebenenfalls nach destillativer Trennung in Fraktionen mit gleicher oder ähnlicher C-Zahl. Ebenfalls können-Olefine oder Olefingemische, die durch Fischer-Tropsch-Synthese erzeugt werden, eingesetzt werden, sowie Olefine, die durch Oligomerisierung von Ethen erhalten werden oder die über Methathesereaktionen oder Telomerisationsreaktion zugänglich sind.

Bevorzugte Edukte sind allgemein α-Olefine wie Propen, 1-Buten, 2-Buten, 1-Hexen, 1-Octen, sowie Dimere und Trimere des Butens (Dibuten, Di-n-buten. Di-iso-buten. Tributen).

Die Hydroformylierung kann kontinuierlich oder diskflntinuierlich durchgeführt werden. Beispiele für technische Ausführungen sind Rührkessel, Blasensäulen, Strahldüsenreaktoren, Rohrreaktoren, oder Schlaufenreaktoren, die zum Teil kaskadiert und/oder mit Einbauten versehen sein können.

Die Reaktion kann durchgehend oder in mehreren Stufen erfolgen. Die Trennung der entstandenen Aldehydverbindungen und des Katalysators kann durch eine herkömmliche Methode, wie Fraktionierung, durchgeführt werden. Technisch kann dies beispielsweise über eine Destillation, über einen Fallfilmverdampfer oder einen Dünnschichtverdampfer erfolgen. Die gilt besonders, wenn der Katalysator in einem hochsiedenden Lösungsmittel gelöst von den niedriger siedenden Produkten abgetrennt wird. Die abgetrennte Katalysatorlösung kann für weitere Hydroformylierungen verwendet werden. Bei Einsatz niederer Olefine (z. B. Propen, Buten, Penten) ist auch ein Austrag der Produkte aus dem Reaktor über die Gasphase möglich.

Die folgenden Beispiele veranschaulichen die vorliegende Erfindung.

Bei allen Beispielen wurde mit Standard-Schlenk-Technik unter Schutzgas gearbeitet. Die Lösungsmittel wurden vor Gebrauch über geeigneten Trocknungsmitteln getrocknet.

### Chlorverbindung A

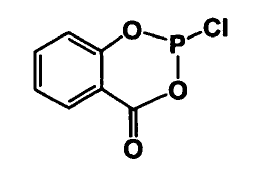

Chlorverbindung A (2-Chlor-4H-1,3,2-benzodioxaphosphorin-4-on) wurde von Fa. Aldrich. Taufkirchen bezogen und verwendet wie geliefert. Chlorverbindung B

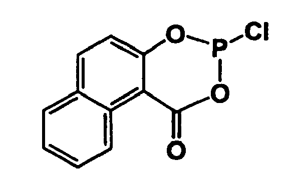

Die Darstellung der Chlorverbindung B erfolgte ausgehend von 2-Hydroxy-1-naphthalincarbonsäure in Anlehnung an BE 667036. Farbwerke Hoechst AG, 1966 *:Chem. Abstr.* 65 (1966) 13741d. Die nachfolgende Synthesebeschreibung verdeutlicht das Vorgehen:

### Umsetzung von 2-Hydroxy-1-naphthalincarbonsäure mit Phosphortrichlorid

In einem sekurierten 250 ml Schlenkrohr werden 9.22 g (0.049 mol) 2-Hydroxy-1-naphthalincarbonsäure, 200 ml getrocknetes Toluol und 0.48 g (0.005 mol) N-Methyl-2-pyrrolidinon vorgelegt. Zu dieser Mischung wird unter Rühren langsam 10.14 g (0.073 mol) Phosphortrichlorid zugegeben. Nach Anschluß des Schlenkrohres an eine Abgasleitung mit einem Gasdurchflußmesser wird die Reaktionsmischung vorsichtig auf 95 °C erhitzt und 5 h bei dieser Temperatur gehalten. Zur Aufarbeitung wird die Reaktionsmischung filtriert und das Lösemittel des Filtrats im Vakuum entfernt.
Ausbeute : 11.01 g (44.6 mmol), entsprechend 91.0 % der Theorie.
³¹P-NMR (Toluol-D₈) : δ 150.9 ppm

### Synthese der Phosphitverbindung (A)

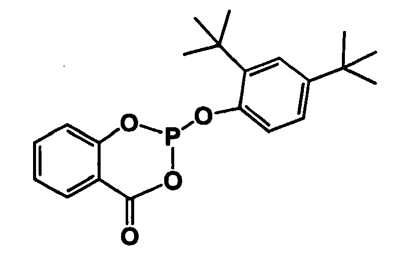

4.5 g 2,4-Di-*tert*-butylphenol (21.81 mmol) werden in 100 ml THF gelöst. Zu dieser Lösung tropft man bei -20°C 13.6 ml einer 1.6 mmol Lösung von *n-*Butyllithium in Hexan (21.81 mmol). Die erhaltene Lithiumphenolatlösung wird bei 0°C langsam zu 32.8 ml einer 0.665 *M* Lösung der Chlorverbindung A (21.81 mmol) in THF gegeben und die resultierende Mischung anschließend 1 h am Rückfluß gekocht. Nach Entfernen des Lösungsmittels im Vakuum erfolgt die Zugabe von 100 ml Hexan und Filtration. Entfernen des Lösungsmittels im Vakuum ergibt ein öliges Produkt.

Ausbeute : 6.58 g (17.669 mmol), entsprechend 81.2 % der Theorie
Elementaranalyse (ber. für C₂₁H₂₅O₄P : M = 372.39 g/Mol C 68.05 (67.73) : H 6.96 (6.77) : P 7.98 (8.32) %
³¹P-NMR (Toluol-D₈) : δ 119.8 ppm. EI-MS (70 eV) : 372 (38 %, M'), 357 (100 %)

### Synthese der Phosphitverbindung (J)

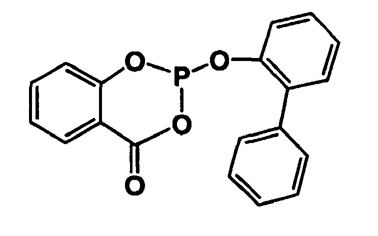

3.617 g 2-Phenylphenol (21.25 mmol) werden in 100 ml THF gelöst. Zu dieser Lösung tropft man bei -20°C 13.28 ml einer 1.6 *M* Lösung von *n*-Butyllithium in Hexan (21.25 mmol). Die erhaltene Lithiumphenolatlösung wird bei 0°C langsam zu 32.0 ml einer 0.665 *M* Lösung der Chlorverbindung A (21.25 mmol) in THF gegeben und die resultierende Mischung anschließend 1 h am Rückfluß gekocht. Nach Entfernen des Lösungsmittels im Vakuum erfolgt die Zugabe von 80 ml Toluol und Filtration. Das Filtrat wird im Vakuum vollständig vom Lösungsmittel befreit. Es wird ein öliges Produkt erhalten.

Ausbeute : 5.80 g (17.24 mmol). entsprechend 81.7 % der Theorie
Elementaranalyse (ber. für C₁₉H₁₃O₄P , M = 336.28 g/Mol ) : C 68.24 (67.86) : H 4.02 (3.90) ; P 9.66 (9.21) %
³¹P-NMR (CD₂Cl₂) : δ 118.4 ppm. EI-MS (70 eV) : 335 (42 %, M⁺). 167 (100 %. M⁺- OC₆H₄-O-C₆H₅)

### Synthese der Phosphitverbindung (D)

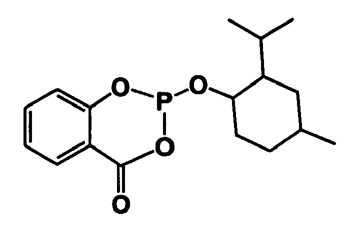

4.155 g Menthol (26.59 mmol) werden in THF (100 ml) gelöst und unter Argon bei -20 °C mit 16.6 ml einer 1.6 *M* Lösung von n-Butyllithium in n-Hexan (26.59 mmol) versetzt. Die Lithiummenthylatlösung wird nach Erwärmen auf Raumtemperatur zu einer auf 0 °C gekühlten Lösung von 5.385 g der Chlorphosphorverbindung A (26.59 mmol) in THF (30 ml) gegeben. Die Reaktionsmischung wird abschließend 1 h am Rückfluss erhitzt, im Vakuum zur Trockne eingeengt und in 100 ml Hexan aufgenommen. Filtration und Entfernen des Lösungsmittels im Vakuum ergeben das Produkt als hellgelbes Öl.
Ausbeute : 6.48 g (20.1 mmol) entsprechend 75 % der Theorie.
³¹P-NMR (CD₂Cl₂) : δ 127.4 ppm . EI-MS (70 eV) m/e = 323 (2 %. *M*⁺+H) : 167 (33 %) : 138 (92 %) : 83 (100 %).
Hydroformylierung von 1-Octen und Di-n-Buten unter Verwendung der Phosphitverbindungen (A). (J) und (D)

Die Hydroformylierungsversuche wurden in einem mit Druckkonstanthaltung, Gasflussmessung. Begasungsrührer und Druckpipette ausgestatteten 200 ml Autoklaven der Fa. Buddeberg, Mannheim, durchgeführt. In dem Autoklaven wurden unter Argonatmosphäre 10 ml einer Lösung des Rhodiums in Form von [Rh(1,5-cyclooctadien)acac] (acac = Acetylacetonat-Anion) (0,604 mM bei 1-Octen bzw. 6,04 mM bei Di-n-Buten) als Katalysatorvorstufe mit der entsprechenden Menge der in Toluol gelösten Phosphitverbindungen gemischt und mit Toluol auf 41 ml aufgefüllt. In die Druckpipette wurden 15 ml 1-0cten bzw. Di-*n*-Buten eingespeist. Nach Austausch der Argonatmosphäre durch Spülen mit Synthesegas (CO/H2 1:1) wurde das Rhodium-Ligand-Gemisch unter Rühren (1500 U/min) bei einem Synthesegasdruck von 30-33 bar bei 1-Octen bzw. 11-13 bar bei Di-*n*-Buten auf 100 °C bei 1-0cten bzw. 120°C bei Di-n-Buten aufgeheizt. Bei Erreichen der gewünschten Reaktionstemperatur wurde der Synthesegasdruck auf 50 bar bei 1-0kten bzw. 20 bar bei Di-n-Buten erhöht und Olefin zugegeben. Die Reaktion wurde bei konstantem Druck (Nachdruckregler der Fa. Bronkhorst (NL)) über 3 h bei 1-Okten bzw. 6 h bei Di-n-Buten geführt. Der Autoklav wurde nach Ablauf der Versuchszeit auf Raumtemperatur abgekühlt. entspannt und mit Argon gespült. Jeweils 1 ml der Autoklavenlösung bei 1-Okten bzw. 2 ml bei Di-n-Buten wurden mit 5 ml n-Pentan versetzt und gaschromatographisch analysiert.
Versuchsparameter für 1-Octen:
Rhodiumkonzentration = 14 ppm, Verhältnis Rh : Ligand : 1-Octen = 1 : 10 : 15700: T= 100 °C. p= 50 bar Synthesegas (CO/H₂ = 1 : 1), t= 3 h. Lösungsmittel : Toluol
Versuchsparameter für Di-*n*-Buten :
Rhodiumkonzentration = 140 ppm. Verhältnis Rh : Ligand : Di-n-Buten = 1 : 10 : 1570: T= 130°C, p= 20 bar Synthesegas (CO/H₂ = 1 : 1). t=
6 h. Lösungsmittel : Toluol

**Tabelle: Hydroformylierung von 1-Octen und D-n-Buten**

| | Phosphit (A) | Phosphit (J) | Phosphit (D) |
|---|---|---|---|
| *1-Octen* | | | |
| Ausbeute (Mol%) | 91 | 43 | 85 |
| *n*-Selektivität(Mol%) | 67.5 | 76.4 | 69.0 |
| *Di-n-Buten* | | | |
| Ausbeute (Mol%) | 42 | 8 | 26 |
| *n*-Selektivität (Mol%) | 49.8 | 43.8 | 38.5 |

Unter der Ausbeute ist die Gesamtausbeute an C₉-Aldehyden zu verstehen. Mit n-Selektivität wird das Verhältnis von endständig zu innenständig hydroformylierten C₉-Aldehyden bezeichnet.

## Patentansprüche

1. Phosphit gemäß Formel I 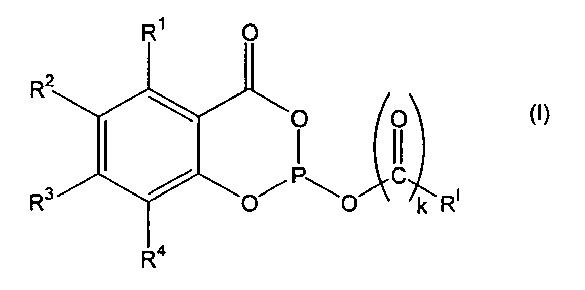 wobei R¹ ausgewählt ist aus einwertigen unsubstituierten aliphatischen, alicyclischen, gemischt aliphatisch-alicyclischen, gemischt aliphatisch-aromatischen, heterocyclischen, und gemischt aliphatisch-heterocyclischen Kohlenwasserstoffresten mit 1 bis 50 Kohlenstoffatomen, oder aus einwertigen aromatischen Kohlenwasserstoffresten mit 1 bis 50 Kohlenstoffatomen, die unsubstituiert oder mit mindestens einem Rest, ausgewählt aus alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 25 Kohlenstoffatomen, F, Cl, Br, I, -CF₃, -CH₂(CF₂)ⱼCF₃ mit j = 0 - 9, -OR⁹, -COR⁹, -CO₂R⁹, -CO₂M. -SR⁹, -SO₂R⁹, -SOR⁹, -SO₃R⁹, -SO₃M, -SO₂NR⁹R¹⁰, -NR⁹R¹⁰, oder -N=CR⁹R¹⁰, substituiert sind, oder aus einwertigen heteroaromatischen Kohlenwasserstoffresten mit 1 bis 50 Kohlenstoffatomen, die unsubstituiert oder mit mindestens einem Rest, ausgewählt aus aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 25 Kohlenstoffatomen, F. Cl, Br, I, -CF₃, -CH₂(CF₂)ⱼCF₃ mit j = 0 - 9, -OR⁹, -COR⁹, -CO₂R⁹, -CO₂M. -SR⁹, -SO₂R⁹, -SOR⁹, -SO₃R⁹, -SO₃M, -SO₂NR⁹R¹⁰, -NR⁹R¹⁰, oder -N=CR⁹R¹⁰, substituiert sind,
R¹, R², R³, und R⁴ jeweils unabhängig voneinander ausgewählt sind aus einwertigen substituierten oder unsubstituierten aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 50 Kohlenstoffatomen, H, F, Cl, Br, I, -CF₃, -CH₂(CF₂)ⱼCF₃ mit j = 0 - 9, -OR⁹, -COR⁹, -CO₂R⁹, -CO₂M, -SR⁹, -SO₂R⁹, -SOR⁹, -SO₃R⁹, -SO₃M. -SO₂NR⁹R¹⁰, -NR⁹R¹⁰, -N=CR⁹R¹⁰, wobei R⁹ und R¹⁰ unabhängig voneinander aus H, einwertigen substituierten oder unsubstituierten aliphatischen und aromatischen Kohlenwasserstoffresten mit 1 bis 25 Kohlenstoffatomen ausgewählt sind und M ein Alkalimetall-, formal ein halbes Erdalkalimetall-. Ammonium- oder Phosphoniumion ist,
oder benachbarte Reste R¹ bis R⁴ zusammen ein kondensiertes substituiertes oder unsubstituiertes aromatisches, heteroaromatisches, aliphatisches, gemischt aromatisch-aliphatisches oder gemischt heteroaromatisch-aliphatisches Ringsystem ausbilden;
und k = 0 ist,
**dadurch gekennzeichnet, dass**
wenn R¹, R², R³ und R⁴ jeweils ein H-Atom sind und k = 0 ist, R¹ keine Alkyl-, Aryl-, Aralkyl-, Alkenyl-, oder Cycloalkylgruppe ist,
oder Formel A, E, F, G, M oder 0

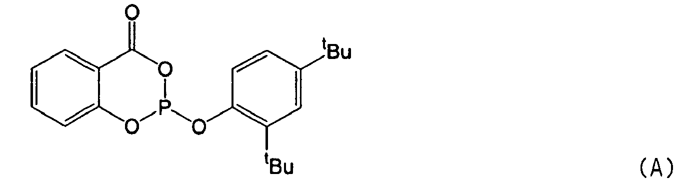

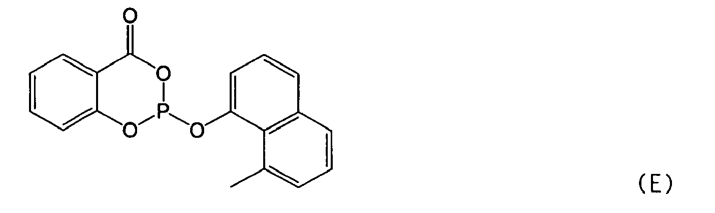

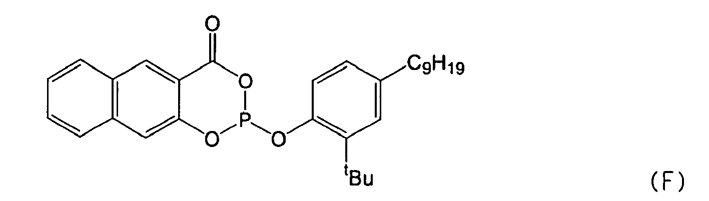

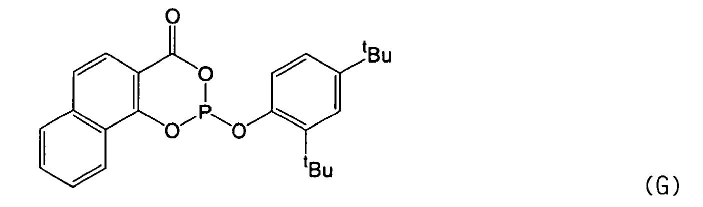

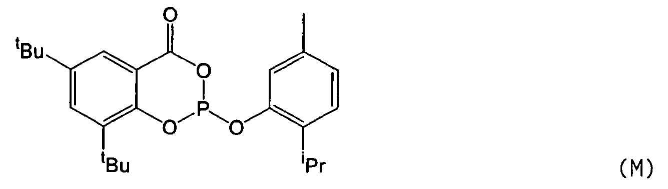

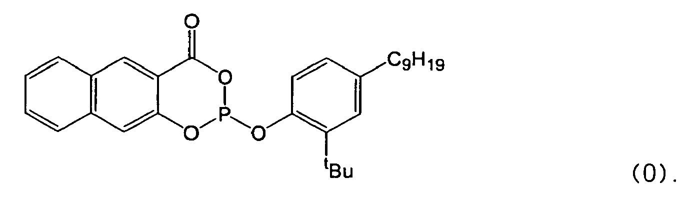

2. Phosphit nach Anspruch 1, wobei der Rest R^{l} des Phosphits ausgewählt ist aus Aromaten oder Heteroaromaten, die ankondensierte aromatische, heteroaromatische und/oder aliphatische Ringe aufweisen, die unsubstituiert oder mit mindestens einem Rest, ausgewählt aus aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 25 Kohlenstoffatomen, F, Cl, Br, I, -CF₃, -CH₂(CF₂)ⱼCF₃ mit j = 0 - 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, NR⁹R¹⁰, oder -N=CR⁹R¹⁰, substituiert sind, wobei R⁹, R¹⁰ und M wie in Anspruch 1 definiert sind.

3. Phosphit nach Anspruch 1 oder 2, wobei benachbarte Reste R¹ bis R⁴ des Phosphits zusammen ein kondensiertes aromatisches, heteroaromatisches, aliphatisches, gemischt aromatisch-aliphatisches oder gemischt heteroaromatisch-aliphatisches Ringsystem ausbilden, das unsubstituiert ist oder mit mindestens einem Rest, ausgewählt aus aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 50 Kohlenstoffatomen, F, Cl, Br, I, -CF₃, -CH₂(CF₂)ⱼCF₃ mit j = 0 - 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰, oder -N=CR⁹R¹⁰, substituiert ist, wobei R⁹, R¹⁰ und M wie in Anspruch 1 definiert sind.

4. Phosphitmetallkomplex, enthaltend ein Metall der 4., 5.. 6.. 7.. 8., 9. oder 10. Gruppe des Periodensystems der Elemente und ein oder mehrere Phosphite gemäß Formel I 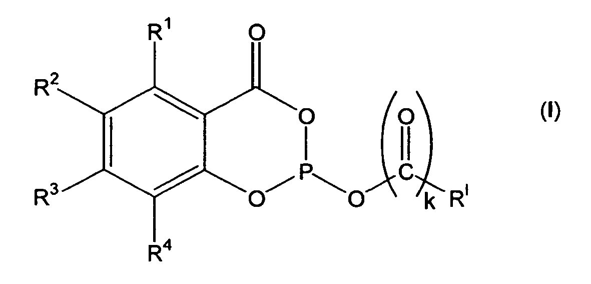 wobei R¹ ausgewählt ist aus einwertigen unsubstituierten aliphatischen, alicyclischen, gemischt aliphatisch-alicyclischen, gemischt aliphatisch-aromatischen, heterocyclischen, und gemischt aliphatisch-heterocyclischen Kohlenwasserstoffresten mit 1 bis 50 Kohlenstoffatomen oder aus einwertigen aromatischen und heteroaromatischen Kohlenwasserstoffresten mit 1 bis 50 Kohlenstoffatomen, die unsubstituiert oder mit mindestens einem Rest, ausgewählt aus aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 25 Kohlenstoffatomen, F, Cl, Br, I, -CF₃, -CH₂(CF₂)ⱼCF₃ mit j = 0 - 9, -OR⁹, -COR⁹, -CO₂R⁹, - CO₂M, -SR⁹, -SO₂R⁹. -SOR⁹, -SO₃R⁹, -SO₃M. -SO₂NR⁹R¹⁰, -NR⁹R¹⁰, oder -N=CR⁹R¹⁰, substituiert sind,
R¹, R², R³, und R⁴ jeweils unabhängig voneinander ausgewählt sind aus einwertigen substituierten oder unsubstituierten aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 50 Kohlenstoffatomen, H. F, Cl. Br, I, -CF₃, -CH₂(CF₂)ⱼCF₃ mit j = 0 - 9, - OR⁹, -COR⁹, -CO₂R⁹, -CO₂M. -SR⁹, -SO₂R⁹, -SOR⁹, -SO₃R⁹, -SO₃M, -SO₂NR⁹R¹⁰. -NR⁹R¹⁰, -N=CR⁹R¹⁰, wobei R⁹ und R¹⁰ unabhängig voneinander aus H, einwertigen substituierten oder unsubstituierten aliphatischen und aromatischen Kohlenwasserstoffresten mit 1 bis 25 Kohlenstoffatomen ausgewählt sind und M ein Alkalimetall-, formal ein halbes Erdalkalimetall-. Ammonium- oder Phosphoniumion ist,
oder benachbarte Reste R¹ bis R⁴ zusammen ein kondensiertes substituiertes oder unsubstituiertes aromatisches, heteroaromatisches, aliphatisches, gemischt aromatisch-aliphatisches oder gemischt heteroaromatisch-aliphatisches Ringsystem ausbilden:
und k = 0 ist.

5. Phosphitmetallkomplex nach Anspruch 4, wobei der Rest R^{l} des Phosphits ausgewählt ist aus Aromaten oder Heteroaromaten, die ankondensierte aromatische, heteroaromatische und/oder aliphatische Ringe aufweisen, die unsubstituiert oder mit mindestens einem Rest, ausgewählt aus aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 25 Kohlenstoffatomen, F, Cl, Br, I, -CF₃, -CH₂(CF₂)ⱼCF₃ mit j = 0 - 9, -OR⁹, -COR⁹, - CO₂R⁹ , -CO₂M, -SR⁹, -SO₂R⁹, -SOR⁹, -SO₃R⁹, -SO₃M, - SO₂NR⁹R¹⁰ , -NR⁹R¹⁰. oder -N=CR⁹R¹⁰. substituiert sind, wobei R⁹, R¹⁰ und M wie in Anspruch 1 definiert sind.

6. Phosphitmetallkomplex nach Anspruch 4 oder 5, wobei benachbarte Reste R¹ bis R⁴ des Phosphits zusammen ein kondensiertes aromatisches, heteroaromatisches, aliphatisches, gemischt aromatisch-aliphatisches oder gemischt heteroaromatisch-aliphatisches Ringsystem ausbilden, das unsubstituiert ist oder mit mindestens einem Rest, ausgewählt aus aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 50 Kohlenstoffatomen, F. Cl, Br, I, -CF₃, -CH₂(CF₂)ⱼCF₃ mit j = 0 - 9, -OR⁹, -COR⁹, -CO₂R⁹. -CO₂M, -SR⁹, -SO₂R⁹, -SOR⁹. -SO₃R⁹, -SO₃M. -SO₂NR⁹R¹⁰, -NR⁹R¹⁰, oder -N=CR⁹R¹⁰, substituiert ist, wobei R⁹, R¹⁰ und M wie in Anspruch 1 definiert sind.

7. Phosphitmetallkomplex nach einem der Ansprüche 4 bis 6, wobei das Metall Rhodium, Platin, Palladium, Kobalt oder Ruthenium ist.

8. Verwendung eines Phosphits wie in einem der Ansprüche 4 bis 6 definiert oder eines Phosphitmetallkomplexes nach einem der Ansprüche 4 bis 7 in der Katalyse.

9. Verwendung eines Phosphits wie in einem der Ansprüche 4 bis 6 definiert oder eines Phosphitmetallkomplexes nach einem der Ansprüche 4 bis 7 in der homogenen Katalyse.

10. Verwendung eines Phosphits wie in einem der Ansprüche 4 bis 6 definiert oder eines Phosphitmetallkomplexes nach einem der Ansprüche 4 bis 7 in einem Verfahren zur Hydroformylierung von Olefinen.

11. Verwendung nach Anspruch 10, wobei weitere phosphorhaltige Liganden anwesend sind.

12. Verfahren zur Hydroformylierung von Olefinen, umfassend die Umsetzung eines Monoolefins oder Monoolefingemisches mit einem Gemisch aus Kohlenmonoxid und Wasserstoff in Gegenwart eines Phosphits wie in einem der Ansprüche 4 bis 6 definiert oder eines Phosphitmetallkomplexes nach einem der Ansprüche 4 bis 7.

13. Verfahren zur Herstellung eines Phosphits nach einem der Ansprüche 1 bis 3, umfassend
Umsetzung einer α-Hydroxyarylcarbonsäure gemäß Formel (1) 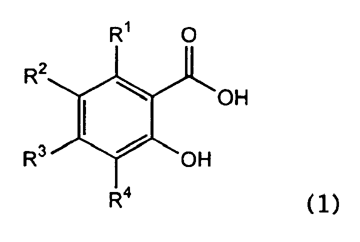 mit PCl₃, PBr₃ oder PI₃ in Gegenwart einer Base unter Ausbildung eines Halogendioxaphosphorinons gemäß Formel (2), wobei Hal = Cl, Br oder I. und 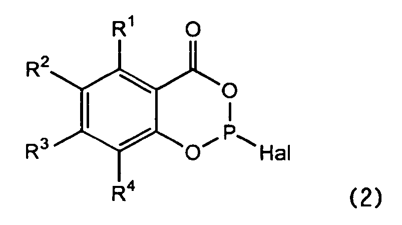 (b) Umsetzung des Halogendioxaphosphorinons (2) in Gegenwart einer Base mit einem Alkohol HO-R^{l}, um ein Phosphit gemäß Formel (I) mit k = 0 zu erhalten,
wobei R¹ bis R⁴, und R¹ wie in Anspruch 1 definiert sind.

14. Verfahren zur Herstellung eines Phosphitmetallkomplexes nach einem der Ansprüche 4 bis 7, umfassend die Umsetzung eines Metalls der 4., 5., 6., 7., 8., 9. oder 10. Gruppe des Periodensystems in elementarer Form oder in Form einer chemischen Verbindung mit einem Phosphit wie in einem der Ansprüche 4 bis 6 definiert.

## Claims

1. A phosphite of the formula I 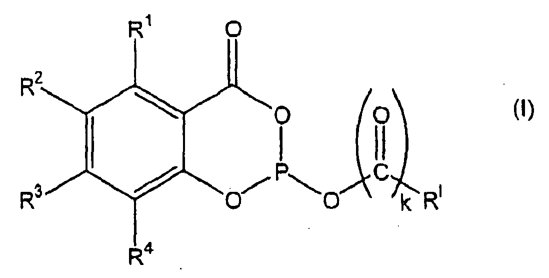 where R' is selected from among monovalent unsubstituted aliphatic, alicyclic, mixed aliphatic-alicyclic, mixed aliphatic-aromatic, heterocyclic and mixed aliphatic-heterocyclic hydrocarbon radicals having from 1 to 50 carbon atoms or from among monovalent aromatic hydrocarbon radicals which have from 1 to 50 carbon atoms and are unsubstituted or substituted by at least one radical selected from among alicyclic, aromatic, heteroaromatic, mixed aliphatic-alicyclic, mixed aliphatic-aromatic, heterocyclic, mixed aliphatic-heterocyclic hydrocarbon radicals having from 1 to 25 carbon atoms, F, C1, Br, I, -CF₃, -CH₂ (CF₂)ⱼCF₃ where j = 0-9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰ and -_{N=CR}⁹_{C}10 or from among monovalent heteroaromatic hydrocarbon radicals which have from 1 to 50 carbon atoms and are unsubstituted or substituted by at least one radical selected from among aliphatic, alicyclic, aromatic, heteroaromatic, mixed aliphatic-alicyclic, mixed aliphatic-aromatic, heterocyclic, mixed aliphatic-heterocyclic hydrocarbon radicals having from 1 to 25 carbon atoms, F, Cl, Br, I, -CF₃, -CH₂(CF₂)ⱼCF₃ with j = 0-9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰ and -N=CR⁹C¹⁰,
R¹, R², R³ and R⁴ are each selected independently from among monovalent substituted or unsubstituted aliphatic, alicyclic, aromatic, heteroaromatic, mixed aliphatic-alicyclic, mixed aliphatic-aromatic, heterocyclic, mixed aliphatic-heterocyclic hydrocarbon radicals having from 1 to 50 carbon atoms, H, F, Cl, Br, I, -CF₃, -CH₂ (CF₂)ⱼCF₃ where j = 0-9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰, -N=CR⁹R¹⁰, where R⁹ and R¹⁰ are selected independently from among H, monovalent substituted or unsubstituted aliphatic and aromatic hydrocarbon radicals having from 1 to 25 carbon atoms and M is an alkali metal ion, formally half an alkaline earth metal ion, an ammonium ion or phosphonium ion,
or adjacent radicals R¹ to R⁴ together form a fused substituted or unsubstituted aromatic, heteroaromatic, aliphatic, mixed aromatic-aliphatic or mixed heteroaromatic-aliphatic ring system;
and k = 0,
**characterized in that**
when R¹, R², R³ and R⁴ are each an H atom and k = 0, R' is not an alkyl, aryl, aralkyl, alkenyl or cycloalkyl group,
or formula A, E, F, G, M or 0

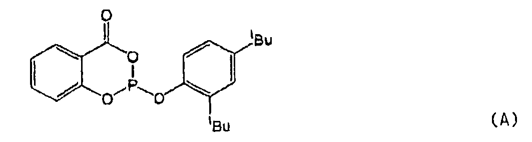

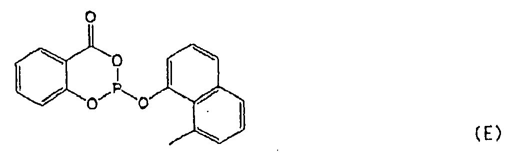

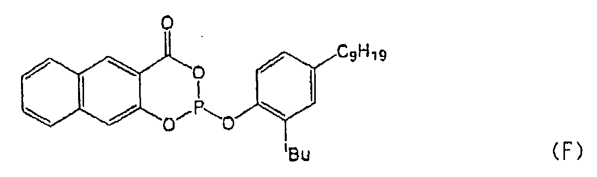

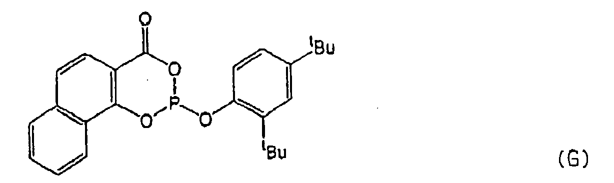

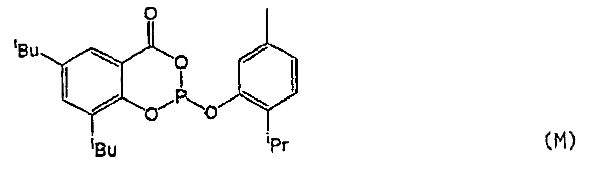

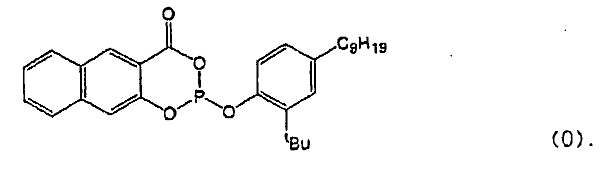

2. A phosphite according to claim 1, wherein the radical R' of the phosphite is selected from among aromatics and heteroaromatics which have fused-on aromatic, heteroaromatic and/or aliphatic rings which are unsubstituted or substituted by at least one radical selected from among aliphatic, alicyclic, aromatic, heteroaromatic, mixed aliphatic-alicyclic, mixed aliphatic-aromatic, heterocyclic, mixed aliphatic-heterocyclic hydrocarbon radicals having from 1 to 25 carbon atoms, F, Cl, Br, I, -CF₃, -CH₂ (CF₂)ⱼCF₃ where j = 0-9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰ or -N=CR⁹R¹⁰, where R⁹, R¹⁰ and M are as defined in claim 1.

3. A phosphite according to either of claims 1 and 2, wherein adjacent radicals R¹ to R⁴ of the phosphite together form a fused aromatic, heteroaromatic, aliphatic, mixed aromatic-aliphatic or mixed heteroaromatic-aliphatic ring system which is unsubstituted or is substituted by at least one radical selected from among aliphatic, alicyclic, aromatic, heteroaromatic, mixed aliphatic-alicyclic, mixed aliphatic-aromatic, heterocyclic, mixed aliphatic-heterocyclic hydrocarbon radicals having from 1 to 50 carbon atoms, F, Cl, Br, I, -CF₃, -CH₂(CF₂)ⱼCF₃ where j = 0-9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰ or -N=CR⁹R¹⁰, where R⁹, R¹⁰ and M are as defined in claim 1.

4. A phosphite-metal complex containing a metal of group 4, 5, 6, 7, 8, 9 or 10 of the Periodic Table of the Elements and one or more phosphites of the formula I 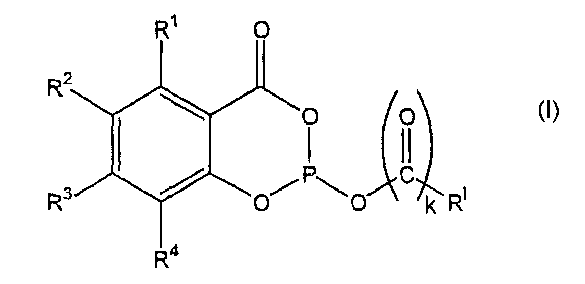 where R' is selected from among monovalent unsubstituted aliphatic, alicyclic, mixed aliphatic-alicyclic, mixed aliphatic-aromatic, heterocyclic and mixed aliphatic-heterocyclic hydrocarbon radicals having from 1 to 50 carbon atoms or from among monovalent aromatic and heteroaromatic hydrocarbon radicals which have from 1 to 50 carbon atoms and are unsubstituted or substituted by at least one radical selected from among aliphatic, alicyclic, aromatic, heteroaromatic, mixed aliphatic-alicyclic, mixed aliphatic-aromatic, heterocyclic, mixed aliphatic-heterocyclic hydrocarbon radicals having from 1 to 25 carbon atoms, F, Cl, Br, I, -CF₃, -CH₂ (CF₂)ⱼCF₃ where j = 0-9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, NR⁹ R¹⁰ and -N=CR⁹R¹⁰,
R¹, R², R³ and R⁴ are each selected independently from among monovalent substituted or unsubstituted aliphatic, alicyclic, aromatic, heteroaromatic, mixed aliphatic-alicyclic, mixed aliphatic-aromatic, heterocyclic, mixed aliphatic-heterocyclic hydrocarbon radicals having from 1 to 50 carbon atoms, H, F, Cl, Br, I, -CF₃, -CH₂(CF₂)ⱼCF₃ where j = 0-9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰, -N=CR⁹R¹⁰, where R⁹ and R¹⁰ are selected independently from among H, monovalent substituted or unsubstituted aliphatic and aromatic hydrocarbon radicals having from 1 to 25 carbon atoms and M is an alkali metal ion, formally half an alkaline earth metal ion, an ammonium ion or phosphonium ion,
or adjacent radicals R¹ to R⁴ together form a fused substituted or unsubstituted aromatic, heteroaromatic, aliphatic, mixed aromatic-aliphatic or mixed heteroaromatic-aliphatic ring system; and k = 0.

5. A phosphite-metal complex according to claim 4, wherein the radical R' of the phosphite is selected from among aromatics and heteroaromatics which have fused-on aromatic, heteroaromatic and/or aliphatic rings which are unsubstituted or substituted by at least one radical selected from among aliphatic, alicyclic, aromatic, heteroaromatic, mixed aliphatic-alicyclic, mixed aliphatic-aromatic, heterocyclic, mixed aliphatic-heterocyclic hydrocarbon radicals having from 1 to 25 carbon atoms, F, Cl, Br, I, -CF₃, -CH₂(CF₂)ⱼCF₃ where j = 0-9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰ or -N=CR⁹R¹⁰, where R⁹, R¹⁰ and M are as defined in claim 1.

6. A phosphite-metal complex according to either of claims 4 and 5, wherein adjacent radicals R¹ to R⁴ of the phosphite together form a fused aromatic, heteroaromatic, aliphatic, mixed aromatic-aliphatic or mixed heteroaromatic-aliphatic ring system which is unsubstituted or is substituted by at least one radical selected from among aliphatic, alicyclic, aromatic, heteroaromatic, mixed aliphatic-alicyclic, mixed aliphatic-aromatic, heterocyclic, mixed aliphatic-heterocyclic hydrocarbon radicals having from 1 to 50 carbon atoms, F, Cl, Br, I, -CF₃, -CH₂(CF₂)ⱼCF₃ where j = 0-9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰ or -NR⁹R¹⁰, -N=CR⁹R¹⁰, where R⁹, R¹⁰ and M are defined as in claim 1.

7. A phosphite metal complex according to any one of claims 4 to 6, wherein the metal is rhodium, platinum, palladium, cobalt or ruthenium.

8. The use of a phosphite as defined in any one of claims 4 to 6 or a phosphite-metal complex according to any one of claims 4 to 7 in catalysis.

9. The use of a phosphite as defined in any one of claims 4 to 6 or a phosphite-metal complex according to any one of claims 4 to 7 in homogenous catalysis.

10. The use of a phosphite as defined in any one of claims 4 to 6 or a phosphite metal complex according to any one of claims 4 to 7 in a process for the hydroformylation of olefins.

11. The use according to claim 10, wherein further phosphorus-containing ligands are present.

12. A process for the hydroformylation of olefins, which comprises reacting a monoolefin or monoolefin mixture with a mixture of carbon monoxide and hydrogen, in the presence of a phosphite as defined in any one of claims 4 to 6 or a phosphite-metal complex according to any one of claims 4 to 7.

13. A process for preparing a phosphite according to any of claims 1 to 3, which comprises
reacting an α-hydroxyarylcarboxylic acid of the formula (1) 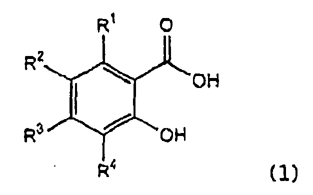 with PCl₃ PBr₃ or PI₃ in the presence of a base to form a halodioxaphosphorinone of the formula (2), where Hal = Cl, Br or I, and 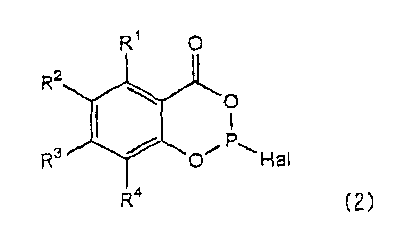 (b) reacting the halodioxaphosphorinone (2) in the presence of a base with
an alcohol HO-R' to give a phosphite of the formula (I) in which k = 0,
where R¹ to R⁹ and R' are as defined in claim 1.

14. A process for preparing a phosphite-metal complex according to any one of claims 4 to 7, which comprises reacting a metal of group 4, 5, 6, 7, 8, 9 or 10 of the Periodic Table in elemental form or in the form of a chemical compound with a phosphite as defined in any one of claims 4 to 6.

## Revendications

1. Phosphite selon la formule I 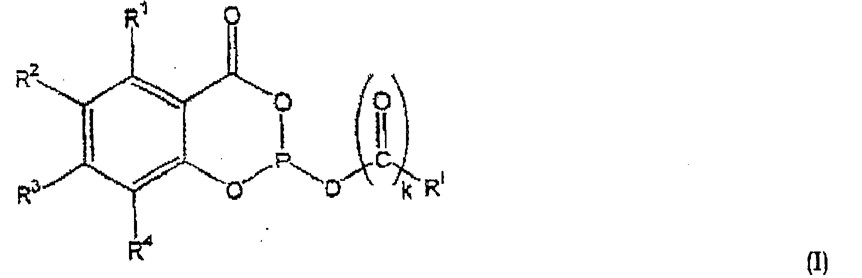 R¹ étant sélectionné parmi des radicaux d'hydrocarbures aliphatiques, alicycliques, aliphatiques-alicycliques mixtes, aliphatiques-aromatiques mixtes, hétérocycliques et aliphatiques-hétérocycliques mixtes non substitués monovalents comprenant 1 à 50 atomes de carbone, ou parmi des radicaux d'hydrocarbures aromatiques monovalents comprenant 1 à 50 atomes de carbone qui ne sont pas substitués ou sont substitués par au moins un radical sélectionné parmi des radicaux d'hydrocarbures alicycliques, aromatiques, hétéroaromatiques, aliphatiques-alicycliques mixtes, aliphatiques-aromatiques mixtes, hétérocycliques, aliphatiques-hétérocycliques mixtes comprenant 1 à 25 atomes de carbone, F, Cl, Br, I, -CF₃, -CH₂(CF₂)jCF₃ avec j = 0 à 9, -OR⁹, -COR⁹, - CO₂R⁹, -CO₂M, -SR⁹, -SO₂R⁹, -SOR⁹, -SO₃R⁹, -SO₃M, -SO₂NR⁹R¹⁰,-NR⁹R¹⁰ ou -N=CR⁹R¹⁰, ou parmi des radicaux d'hydrocarbures hétéroaromatiques monovalents comprenant 1 à 50 atomes de carbone qui ne sont pas substitués ou sont substitués par au moins un radical sélectionné parmi des radicaux d'hydrocarbures aliphatiques, alicycliques, aromatiques, hétéroaromatiques, aliphatiques-alicycliques mixtes, aliphatiques-axomatiques mixtes, hétérocycliques, aliphatiques-hétérocycliques mixtes comprenant 1 à 25 atomes de carbone, F, Cl, Br, I, -CF₃, -CH₂(CF₂)jCF₃ avec j = 0 à 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰ ou -N=CR⁹R¹⁰, R¹, R², R³ et R⁴ étant chacun sélectionnés indépendamment l'un de l'autre parmi des radicaux d'hydrocarbures aliphatiques, alicycliques, aromatiques, hétéroaromatiques, aliphatiques-alicycliques mixtes, aliphatiques-aromatiques mixtes, hétérocycliques, aliphatiques-hétérocycliques mixtes substitués ou non substitués monovalents comprenant 1 à 50 atomes de carbone, H, F, Cl, Br, I, -CF₃, -CH₂(CF₂)jCF₃ avec j = 0 à 9, -OR⁹, -COR⁹, -CO₂R⁹, -CO₂M, -SR⁹, -SO₂R⁹, -SOR⁹, - SO₃R⁹, -SO₃M, -SO₂NR⁹R¹⁰, -NR⁹R¹⁰, -N=CR⁹R¹⁰, R⁹ et R¹⁰ étant sélectionnés indépendamment l'un de l'autre parmi H, des radicaux d'hydrocarbures aliphatiques et aromatiques substitués ou non substitués monovalents comprenant 1 à 25 atomes de carbone et M étant un ion métallique alcalin, de formal alcalin, un demi-ion métallique alcalino-terreux, d'ammonium ou de phosphinium,
ou des radicaux voisins R¹ à R⁴ formant ensemble un système cyclique aromatique, hétéroaromatique, aliphatique, aromatique-aliphatique mixte ou hétéroaromatique-aliphatique mixte substitué ou non substitué condensé ;
et k étant = 0,
**caractérisé en ce que**
lorsque R¹, R², R³ et R⁴ sont chacun un atome de H et k est = O, R¹ n'est pas un groupe alkyle, aryle, aralkyle, alcényle ou cycloalkyle,
ou de formule A, E, F, G, M ou O

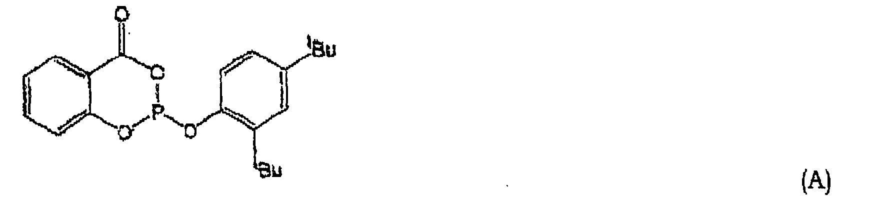

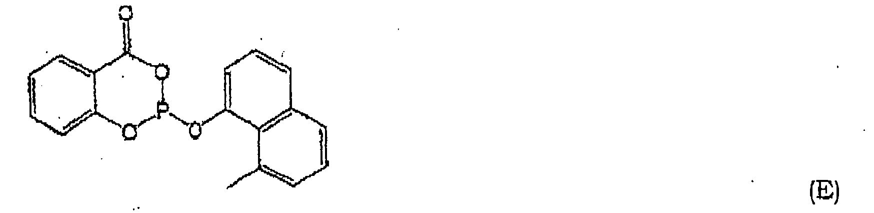

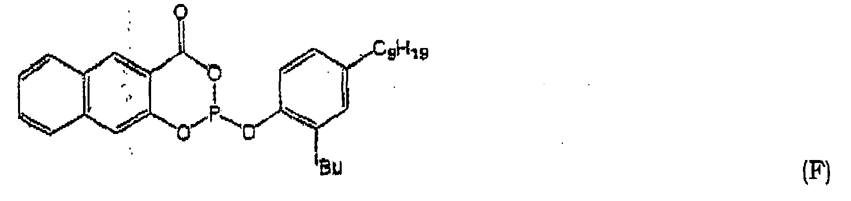

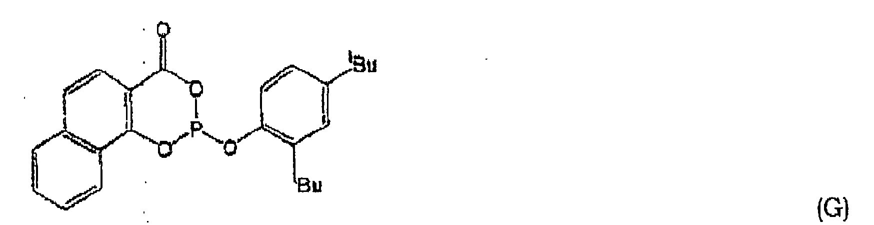

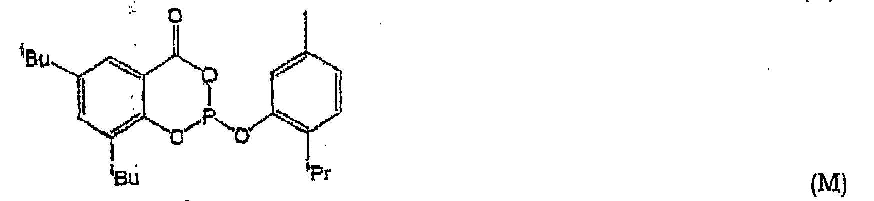

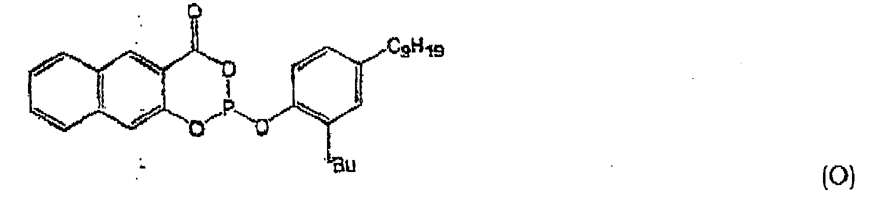

2. Phosphite selon la revendication 1, le radical R¹ du phosphite étant sélectionné parmi des composés aromatiques ou hétéroaromatiques qui présentent des anneaux aromatiques, hétéroaromatiques et/ou aliphatiques condensés qui ne sont pas substitués ou sont substitués par au moins un radical sélectionné parmi des radicaux d'hydrocarbures aliphatiques, alicycliques, aromatiques, hétéroaromatiques, aliphatiques-alicycliques mixtes, aliphatiques-aromatiques mixtes, hétérocycliques, aliphatiques-hétérocy,câques mixtes comprenant 1 à 25 atomes.de carbone, F, Cl, Br, I, -CF₃, -CH₂(CF₂)ⱼCF₃ avec j = 0 à 9, -OR⁹, -COR⁹, - CO₂R⁹, -CO₂M, -SR⁹, -SO₂R⁹, -SOR⁹, -SO₃R⁹, -SO₃M -SO₂NR⁹R¹⁰,-NR⁹R¹⁰ ou -N=CR⁹R¹⁰, R⁹, R¹⁰ et M étant définis comme à la revendication 1.

3. Phosphite selon la revendication 1 ou 2, des radicaux voisins R¹ à R⁴ du phosphite formant ensemble un système cyclique aromatique, hétéroaromatique, aliphatique, aromatique-aliphatique mixte ou hétéroaromatique-aliphatique mixte condensé qui n'est pas substitué ou est substitué par au moins un radical sélectionné parmi des radicaux d'hydrocarbures aliphatiques, alicycliques, aromatiques, hétéroaromatiques, aliphatiques-alicycliques mixtes, aliphatiques-aromatiques mixtes, hétérocycliques, aliphatiques-hétérocycliques mixtes comprenant 1 à 50 atomes de carbone, F, Cl, Br, I, -CF₃, -CH₂(CF₂)ⱼCF₃ avec j = 0 à 9, -OR⁹, -COR⁹, -CO₂R⁹, -CO₂M, -SR⁹, -SO₂R⁹, -SOR⁹, -SO₃R⁹, -SO₃M, - SO₂NR⁹R¹⁰, -NR⁹R¹⁰ ou -N-CR⁹R¹⁰, R⁹, R¹⁰ et M étant définis comme à la revendication 1.

4. Complexe métallique de phosphite contenant un métal du 4^{ème}, 5^{ème}, 6^{ème}, 7^{ème}, 8^{ème}, 9^{ème} ou 10^{ème} groupe de la classification périodique des éléments et un ou plusieurs phosphites selon la formule I 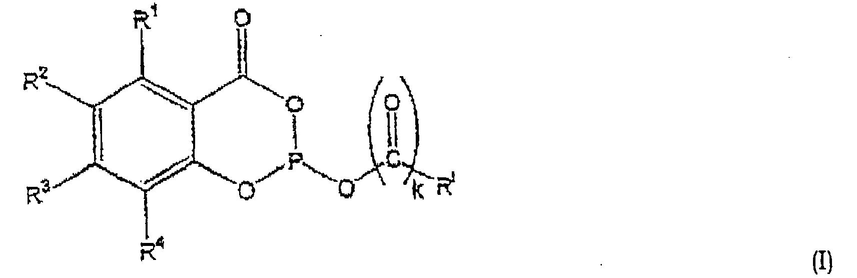 R¹ étant sélectionné parmi des radicaux d'hydrocarbures aliphatiques, alicycliques, aliphatiques-alicycliques mixtes, aliphatiques-aromatiques mixtes, hétérocycliques et aliphatiques-hétérocycliques mixtes non substitués monovalents comprenant 1 à 50 atomes de carbone, ou parmi des radicaux d'hydrocarbures aromatiques et hétéroaromatiques monovalents comprenant 1 à 50 atomes de carbone qui ne sont pas substitués ou sont substitués par au moins un radical sélectionné parmi des radicaux d'hydrocarbures aliphatiques, alicycliques, aromatiques, hétéroaromatiques, aliphatiques-alicycliques mixtes, aliphatiques-aromatiques mixtes, hétérocycliques, aliphatiques-hétérocycliques mixtes comprenant 1 à 25 atomes de carbone, F, Cl, Br, I, -CF₃, CH₂(CF₂)jCF₃ avec j = 0 à 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰ ou -N=CR⁹R¹⁰,
R¹, R², R³ et R⁴ étant chacun sélectionnés indépendamment l'un de l'autre parmi des radicaux d'hydrocarbures aliphatiques, alicycliques, aromatiques, hétéroaromatiques, aliphatiques-alicycliques mixtes, aliphatiques-aromatiques mixtes, hétérocycliques, aliphatiques-hétérocycliques mixtes substitués ou non substitués monovalents comprenant 1 à 50 atomes de carbone, H, F, Cl, Br, I, -CF₃, -CH₂(CF₂)ⱼCF₃ avec j = 0 à 9, -OR⁹, -COR⁹, -CO₂R⁹, -CO₂M, -SR⁹, -SO₂R⁹, -SOR⁹, - SO₃R⁹, -SO₃M, -SO₂NR⁹R¹⁰, -NR⁹R¹⁰, -N=CR⁹R¹⁰, R⁹ et R¹⁰ étant sélectionnés indépendamment l'un de l'autre parmi H, des radicaux d'hydrocarbures aliphatiques et aromatiques substitués ou non substitués monovalents comprenant 1 à 25 atomes de carbone et M étant un ion métallique alcalin, de formal alcalin, un demi-ion métallique alcalino-terreux, d'ammonium ou de phosphinium,
ou des radicaux voisins R¹ à R⁴ formant ensemble un système cyclique aromatique, hétéroaromatique, aliphatique, aromatique-aliphatique mixte ou hétéroaromatique-aliphatique mixte substitué ou non substitué condensé ;
et k étant = 0.

5. Complexe métallique de phosphite selon la revendication 4, le radical R¹ du phosphite étant sélectionné parmi des composés aromatiques ou hétéroaromatiques qui présentent des anneaux aromatiques, hétéroaromatiques et/ ou aliphatiques condensés qui ne sont pas substitués ou sont substitués par au moins un radical sélectionné parmi des radicaux d'hydrocarbures aliphatiques, alicycliques, aromatiques, hétéroaromatiques, aliphatiques-alicycliques mixtes, aliphatiques-aromatiques mixtes, hétérocycliques, aliphatiques-hétérocycliques mixtes comprenant 1 à 25 atomes de carbone, F, Cl, Br, I, -CF₃, -CH₂(CF₂)jCF₃ avec j = 0 à 9, -OR⁹, -COR⁹, -CO₂R⁹, -CO₂M, -SR⁹, -SO₂R⁹, -SOR⁹, -SO₃R⁹, - SO₃M, -SO₂NR⁹R¹⁰, -NR⁹R¹⁰ ou -N=CR⁹R¹⁰, R⁹, R¹⁰ et M étant définis comme à la revendication 1.

6. Complexe métallique de phosphite selon la revendication 4 ou 5, des radicaux voisins R¹ à R⁴ du phosphite formant ensemble un système cyclique aromatique, hétéroaromatique, aliphatique, aromatique-aliphatique mixte ou hétéroaromatique-aliphatique mixte condensé qui n'est pas substitué ou est substitué par au moins un radical sélectionné parmi des radicaux d'hydrocarbures aliphatiques, alicycliques, aromatiques, hétéroaromatiques, aliphatiques-alicycliques mixtes, aliphatiques-aromatiques mixtes, hétérocycliques, aliphatiques-hétérocycliques mixtes comprenant 1 à 50 atomes de carbone, F, Cl, Br, I, -CF₃, - CH₂(CF₂)ⱼCF₃ avec j = 0 à 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰ ou -N=CR⁹R¹⁰, R⁹, R¹⁰ et M étant définis comme à la revendication 1.

7. Complexe métallique de phosphite selon l'une quelconque des revendications 4 à 6, le métal étant le rhodium, le platine, le palladium, le cobalt ou le ruthénium.

8. Utilisation d'un phosphite tel que défini dans l'une quelconque des revendications 4 à 6 ou d'un complexe métallique de phosphite selon l'une quelconque des revendications 4 à 7 dans la catalyse.

9. Utilisation d'un phosphite tel que défini dans l'une quelconque des revendications 4 à 6 ou d'un complexe métallique de phosphite selon l'une quelconque des revendications 4 à 7 dans la catalyse homogène.

10. Utilisation d'un phosphite tel que défini dans l'une quelconque des revendications 4 à 6 ou d'un complexe métallique de phosphite selon l'une quelconque des revendications 4 à 7 dans un procédé en vue de l'hydroformylation d'oléfines.

11. Utilisation selon la revendication 10, d'autres ligands contenant du phosphore étant présents.

12. Procédé en vue de l'hydroformylation d'oléfines, comprenant la conversion d'une mono-oléfine ou d'un mélange de mono-oléfines avec un mélange de monoxyde de carbone et d'hydrogène en présence d'un phosphite tel que défini dans l'une quelconque des revendications 4 à 6
ou d'un complexe métallique de phosphite selon l'une quelconque des revendications 4 à 7.

13. Procédé en vue de la production d'un phosphite selon l'une quelconque des revendications 1 à 3, comprenant
(a) la conversion d'un acide α-hydroxyarylcarboxylique selon la formule (1) 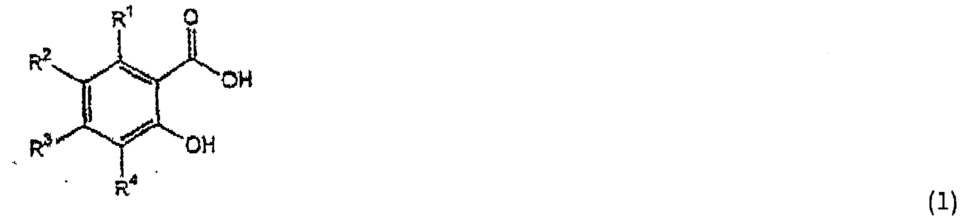 avec PCl₃, PBr₃ ou PI₃ en présence d'une base en formant une halogénodioxaphosphorinone selon la formule (2), Hal = Cl, Br ou I et 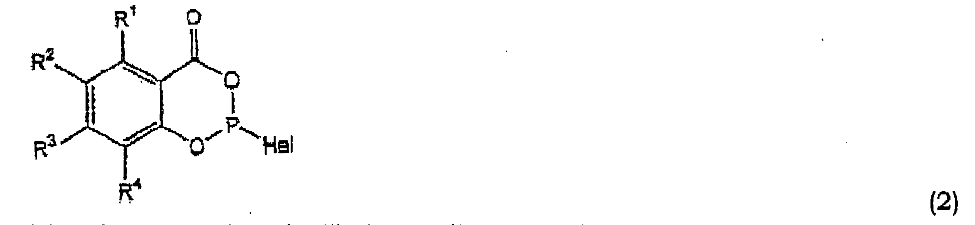
(b) la conversion de l'halogénodioxaphosphorinone (2) en présence d'une base avec
un alcool HO-R¹ afin d'obtenir un phosphite selon la formule (I) avec k = O,
R¹ à R⁴ et R¹ étant définis comme à la revendication 1.

14. Procédé en vue de la production d'un complexe métallique de phosphite selon l'une quelconque des revendications 4 à 7, comprenant la conversion d'un métal du 4^{ème}, 5^{ème}, 6^{ème}, 7^{ème}, 8^{ème}, 9^{ème} ou 10^{ème} groupe de la classification périodique sous forme élémentaire ou sous forme d'un composé chimique avec un phosphite tel que défini dans l'une quelconque des revendications 4 à 6.
